# EUROPEAN PATENT SPECIFICATION

(11) **EP 4 069 643 B1**
(45) Date of publication and mention of the grant of the patent: **19.03.2025**
(21) Application number: 20834026.5
(22) Date of filing: 04.12.2020
(51) Int. Cl.: G02F 1/00, C07D 285/14, G01N 21/64, G01N 33/18, C02F 5/10, C02F 1/00, G01N 21/77

(54) **METHODS AND SYSTEMS FOR MONITORING OR CONTROLLING ANTI-SCALANT CONCENTRATION**
VERFAHREN UND SYSTEME ZUR ÜBERWACHUNG ODER STEUERUNG DER ANTISKALANTKONZENTRATION
PROCÉDÉS ET SYSTÈMES DE SURVEILLANCE OU DE CONTRÔLE DE LA CONCENTRATION ANTI-SCALANTE

(30) Priority: 05.12.2019 US 201962944205 P; 23.01.2020 FI 20205065
(43) Date of publication of application: 12.10.2022
(73) Proprietor: Sterling Specialty Chemicals Holding UK Limited, Middlesex HA1 1BE (GB)
(72) Inventor: NUUTINEN, Vesa, 02270 Espoo (FI); METSÄLÄ, Erkki Johannes, 02270 Espoo (FI); VUORI, Vesa, 02270 Espoo (FI); HESAMPOUR, Mehrdad, 02270 Espoo (FI)
(74) Representative: Heinonen & Co
(86) International application number: PCT/US2020/063282
(87) International publication number: WO 2021/113621

(56) References cited:
- WO-A1-2014/009445
- WO-A1-2019/027608
- JP-A- H05 277 491
- US-B1- 6 280 635

## Description

### Cross-reference to Related Applications

This application claims priority to U.S. Provisional Patent Application No. 62/944,205, filed December 5, 2019, and Finnish Patent Application No. 20205065, filed January 23, 2020.

### Field of the Invention

The present invention relates to monitoring and/or controlling product dosing of anti-scalant compositions.

### Background

Anti-scalants (i.e., scale inhibitors) are used to inhibit scale formation in various fluid treatment applications, such as squeeze treatment, cooling water, thermal desalination, membrane desalination, etc. In some, if not all, of these applications, it is necessary or desirable to monitor a concentration of an anti-scalant in a fluid, such as water, and/or control the product dosing accordingly.

The current fluorescence-based monitoring methods, however, are offline methods, which typically require sample pretreatment and manual feeding of dosing values into a control system. These methods, as a result, are usually laborious, have a relatively low accuracy, or a combination thereof.

There remains a need for methods for monitoring and/or controlling product dosing of anti-scalants that overcome one or more of the foregoing disadvantages.

### Brief Summary

Provided herein are methods of monitoring and/or controlling product dosing that may address one or more of the foregoing disadvantages, including methods that include real time analysis and dosing systems. The present invention relates to methods and systems that permit online measurement of an amount of an anti-scalant, thereby offering better control of a chemical feed. Embodiments of the methods and systems provided herein may reduce operator labor cost, because, for example, automation can reduce the number of operators or shifts required to operate a system or method. Embodiments of the methods and systems provided herein may reduce down time, maintenance costs, or a combination thereof, because, for example, the methods and systems may provide a warning of equipment failure before it occurs. Embodiments of the methods and systems herein may reduce energy costs, because, for example, automation can assist with the monitoring of energy intensive applications.

In one aspect the present invention relates to systems for monitoring and/or controlling dosing of a composition, such as an anti-scalant, are provided. In some embodiments, the systems include an analyzer that includes a fluorometer; a controller that includes a processor and a memory unit; a dosing algorithm stored by the memory unit of the controller; and a pump configured to add an amount of an anti-scalant composition into a fluid stream of a fluid treatment system. In some embodiments, the processor is configured to (i) receive from the analyzer a first signal that includes fluorometry data collected by the fluorometer from an inlet stream and/or an outlet stream of the fluid treatment system, (ii) determine a dosing instruction by applying the fluorometry data to the dosing algorithm, and (iii) provide a second signal that includes the dosing instruction to the pump. In some embodiments, the pump is configured to modify, based on the dosing instruction, the amount of the anti-scalant composition that is added to the fluid stream of the fluid treatment system.

The system of the present invention is defined in claim 1.

In another aspect the present invention relates to methods for monitoring and/or controlling dosing of a composition, such as an anti-scalant, are provided. In some embodiments, the methods include provided a system as described herein; adding with a pump an amount of an anti-scalant composition to a fluid stream to create an inlet stream; analyzing the inlet stream or an outlet stream with a fluorometer to generate fluorometry data that include (i) a first intensity at a fluorescence emission maximum of a fluorescent tracer or a monomer tag at a wavelength pre-selected for the fluorescent tracer or the monomer tag, respectively, (ii) a measured amount of the anti-scalant composition, or (iii) a combination thereof; transferring a first signal that includes the fluorometry data to the processor of a controller; generating with the controller a first dosing instruction by applying the fluorometry data to a dosing algorithm; transferring a second signal that includes the first dosing instruction to the pump; and modifying with the pump, based on the first dosing instruction, an amount of the anti-scalant composition that is added to the fluid stream so that a first modified amount of the anti-scalant composition is added to the fluid stream.

The method of the present invention is defined in claim 6.

Additional aspects will be set forth in part in the description which follows, and in part will be obvious from the description, or may be learned by practice of the aspects described herein. The advantages described herein may be realized and attained by means of the elements and combinations particularly pointed out in the appended claims. It is to be understood that both the foregoing general description and the following detailed description are exemplary and explanatory only and are not restrictive.

### Brief Description of the Drawings

**FIG. 1** depicts an embodiment of a flow through sample cell that may be used in the systems and methods herein.
**FIG. 2** depicts a theoretical plot of anti-scalant dosage relative to a setpoint versus time.
**FIG. 3A** depicts a schematic of an embodiment of a system.
**FIG. 3B** depicts a schematic of an embodiment of a system.
**FIG. 4** is a flow chart depicting elements of an embodiment of a method.
**FIG. 5** depicts an example of a squeeze treatment return profile.

### Detailed Description

The systems and methods provided herein may be used to monitor and/or control dosing of a composition, such as an anti-scalant composition. In some embodiments, the systems and methods provided herein are used to monitor dosing of a composition, such as an anti-scalant composition. In some embodiments, the systems and methods provided herein are used to monitor and control dosing of a composition, such as an anti-scalant composition.

### Systems

The systems provided herein may include an analyzer, a controller, and a pump.

In some embodiments, the analyzer includes a fluorometer. The fluorometer generally may include any device capable of collecting fluorometry data.

In some embodiments, the fluorometry data collected by the fluorometer include (i) an intensity at a fluorescence emission maximum of a fluorescent tracer or a monomer tag at a wavelength pre-selected for the fluorescent tracer or the monomer tag, respectively, (ii) a measured amount of an anti-scalant composition, or (iii) a combination thereof. When the fluorometry data collected by the fluorometer include an intensity at a fluorescence emission maximum of a fluorescent tracer or a monomer tag, the florometry data may include one or more complete, or at least partial, spectra collected by the fluorometer.

In some embodiments, the fluorometry data include an intensity at a fluorescence emission maximum, and such fluorometry data may include a sum of emission intensities. Depending on the quality of an emission peak, one emission value or the sum of several values may be used. If, for example, an emission peak is relatively smooth, then only one intensity value may be included in the fluorometry data, but if the shape of an emission peak is not relatively smooth (e.g., corrugated or otherwise), then the sum of several intensity values may be included in the fluorometry data. As an example, for an anti-scalant composition that includes sodium styrene sulfonate, the sum of emission intensities over the range of about 270 nm to about 310 nm could be recorded.

If two or more different fluorescent tracers or monomer tags are present in an anti-scalant composition, then the fluorometry data may include (i) a first intensity at a first fluorescence emission maximum of a first fluorescent tracer or a first monomer tag at a wavelength pre-selected for the first fluorescent tracer or the first monomer tag, respectively, and (ii) a second intensity at a second fluorescence emission maximum of a second fluorescent tracer or a second monomer tag at a wavelength pre-selected for the second fluorescent tracer or the second monomer tag, respectively.

The fluorometer may measure fluorescence emission in any manner or known method. For example, the fluorometer may measure fluorescence by photodiode in the spectral range of about 450 nm to about 850 nm. Other spectral ranges and methods of detection, e.g., a photomultiplier, also may be used.

In some embodiments, the fluorometer includes one or more optical filters. The one or more optical filters may permit relatively small spectral separation between excitation and emission wavelengths.

In some embodiments, an analyzer determines, based on the first intensity and/or the second intensity, an amount (e.g., concentration) of anti-scalant composition. Therefore, the fluorometry data transferred to a controller from an analyzer may include the amount (e.g., concentration) of anti-scalant composition. In some embodiments, a controller (e.g., a processor of a controller) determines, based on the first intensity and/or the second intensity, an amount (e.g., concentration) of anti-scalant composition. Therefore, the fluorometry data transferred to a controller from an analyzer may not include the amount (e.g., concentration) of anti-scalant composition.

The fluorometer may be configured to collect fluorometry data continuously, intermittently, or a combination thereof. For example, a fluorometer may be configured to collect fluorometry data intermittently at regular intervals, irregular intervals, or a combination thereof.

Any fluorometer may be used in the systems and methods provided herein. Non-limiting examples of fluorometers that may be used in the systems and methods herein include a TRASAR^{®} 3000/8000 fluorometer (Ondeo Nalco Company, Naperville, IL, USA); a HITACHI^{®} F-4500 fluorometer (Hitachi Instruments Inc., San Jose, CA, USA); a JOBIN YVON^{®} FluoroMax-3 "SPEX" fluorometer (JOBIN YVON Inc., Edison, NJ); a GILFORD^{®} Fluoro-IV spectrophotometer (Research Instruments International of San Diego, CA, USA). A commercially available fluorometer may be modified in any manner prior to its use in the systems and methods provided herein.

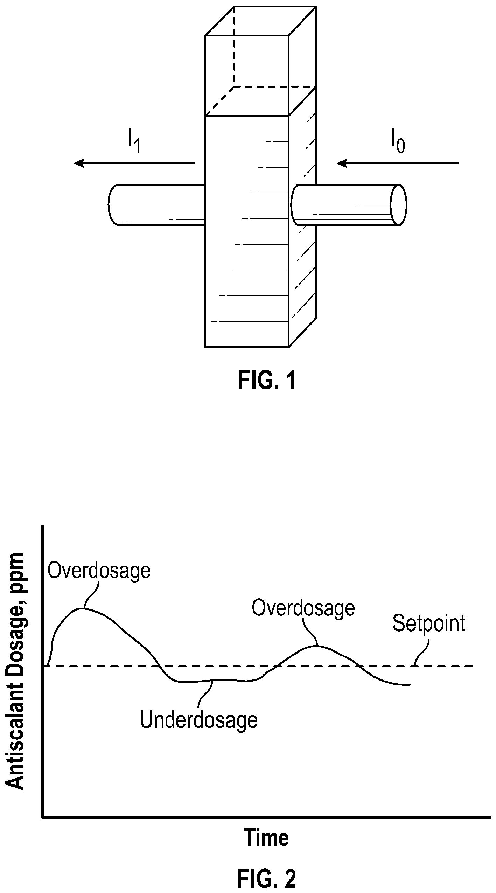

In some embodiments, the analyzer includes a flow through sample cell. An embodiment of a flow through sample cell is depicted at **FIG. 1****.** In some embodiments, the analyzer includes a flow through sample cell, with custom excitations for fluorescence (and scattering) measurement at different wavelength ranges. The different wavelength ranges may include ultraviolet (UV) (e.g., 200-370 nm), or visible and near-infrared (e.g., 370-850 nm) spectral range.

An analyzer may permit sample cells to be cleaned, such as with pressure cycles/bursts. Therefore, the analyzer may provide an automatic cleanup system.

Although a fluorometer is used in embodiments of the systems and methods herein, it should be noted that other instruments, materials, or techniques may be used in addition to, or instead of, a fluorometer or fluorometry, respectively. Non-limiting examples of other analytical techniques include high-pressure liquid chromatography (HPLC)-fluorescence analysis, colorimetry analysis, ion selective electrode analysis, transition metal analysis, or the like. Non-limiting examples of other materials or techniques include a molecular sieve treatment, a pH adjustment, or a combination thereof.

In some embodiments, the analyzer includes a fluorometer and an instrument configured to perform HPLC. The HPLC/fluorometry analysis may permit a fluorescent tracer to be separated from a fluid, such as a fluid of an inlet stream and/or an outlet stream, prior to collecting fluorometry data.

In some embodiments, the analyzer includes molecular sieves, which may be disposed in a reservoir, such as a reservoir of the analyzer. A stream, prior to being analyzed by an analyzer, may be contacted with the molecular sieves, which may remove or reduce an amount of one or more compounds which may interfere with a measurement.

In some embodiments, the controller includes a processor and a memory unit.

In some embodiments, the systems includes a dosing algorithm stored by the memory unit of the controller. A memory unit may store one or more dosing algorithms, and each dosing algorithm may be configured and/or selected in view of one or more parameters, including, but not limited to, the type of fluid treatment system, the type of composition, such as an anti-scalant composition, a desired concentration of a composition, such as an anti-scalant composition, a desired range of permissible deviation from a setpoint, or a combination thereof.

In some embodiments, a dosing algorithm determines, or is based on, a difference between (i) an amount (e.g., concentration) of an anti-scalant composition determined by fluorometry data, and (ii) a setpoint (e.g., a pre-determined, set amount of anti-scalant composition). As depicted, for example, at **FIG. 2****,** the dosing algorithm may be used to devise dosing instructions that prevent the anti-scalant dosage from deviating, to an undesirable extent, from a setpoint.

In some embodiments, the processor is configured to (i) receive from the analyzer a first signal that includes fluorometry data collected by the fluorometer from an inlet stream and/or an outlet stream of the fluid treatment system, (ii) determine a dosing instruction by applying the fluorometry data to the dosing algorithm, and (iii) provide a second signal that includes the dosing instruction to the pump.

In some embodiments, the pump is configured to add an amount of an anti-scalant composition to a fluid stream of a fluid treatment system.

In some embodiments, the pump is configured to modify, based on the dosing instruction, the amount of the anti-scalant composition that is added to the fluid stream of the fluid treatment system. The pump, therefore, may be configured to maintain, increase, and/or decrease the amount of anti-scalant composition that is added to the fluid stream of a fluid treatment system.

The components of the systems provided herein may be configured to communicate in any manner. For example, two or more components of the systems may communicate via a wired connection, wirelessly, a pneumatic connection, or the like. Fluorometry data, dosing instructions, or a combination thereof may be transferred between any two components via a wired or wireless connection.

In some embodiments, the systems provided herein include a reservoir in fluid communication with the pump. An anti-scalant composition may be disposed in the reservoir.

As used herein, two components are in "fluid communication" when the two components are connected directly or indirectly in a manner that permits a fluid of any one or more states (e.g., liquid and/or gas) to be transferred from a first component to a second component, the second component to the first component, or a combination thereof.

In some embodiments, an analyzer is in fluid communication with the outlet stream of the fluid treatment system. For example, the analyzer may be in direct fluid communication with the outlet stream. The outlet stream may be a reject stream of a fluid treatment system. In some embodiments, an analyzer is in fluid communication with an outlet stream via a side stream. For example, a side stream may be drawn from an outlet stream, and an analyzer may be configured to perform a fluorescence measurement of a fluid of the side stream. The outlet stream may include a fluid and an anti-scalant composition.

In some embodiments, an analyzer is in fluid communication with the inlet stream of the fluid treatment system. For example, the analyzer may be in direct fluid communication with the inlet stream. In some embodiments, an analyzer is in fluid communication with an inlet stream via a side stream. For example, a side stream may be drawn from an inlet stream, and an analyzer may be configured to perform a fluorescence measurement of a fluid of the side stream. The inlet stream may include a fluid and an anti-scalant composition.

In some embodiments, the systems and methods herein are configured to analyze an inlet stream of a fluid treatment system, an outlet stream of a fluid treatment system, or an inlet stream and an outlet stream of a fluid treatment system. When the systems and methods herein are configured to analyze an inlet stream and an outlet stream of a fluid treatment system, the systems may include one analyzer that analyzes both the inlet stream and the outlet stream, or a first analyzer and a second analyzer that analyzes the inlet stream and the outlet stream, respectively.

In some embodiments, the fluid treatment system is a water treatment system.

In some embodiments, an anti-scalant composition includes (a) a fluorescent tracer, (b) a tagged polymer comprising a monomer tag, or (c) a combination thereof.

In some embodiments, the systems provided herein include an automated control loop. As used herein, the phrase "automated control loop" refers to a feature of a system that includes measuring a parameter, such as an amount of an anti-scalant composition, and deciding, based on the measurement of the parameter, an action, such as modifying an amount of anti-scalant composition introduced to a system. In some embodiments, an analyzer, a controller, and a pump of the systems provided herein form an automated control loop configured to (i) collect fluorometry data continuously from the outlet stream of the fluid treatment system, (ii) modify continuously with the pump the amount of the anti-scalant composition that is added to the fluid stream of the fluid treatment system, or (iii) a combination thereof. In some embodiments, an analyzer, a controller, and a pump of the systems provided herein form an automated control loop configured to (i) collect fluorometry data intermittently from the outlet stream of the fluid treatment system, (ii) modify intermittently with the pump the amount of the anti-scalant composition that is added to a fluid stream of the fluid treatment system, or (iii) a combination thereof.

In some embodiments, the systems or methods herein include a feedback control loop, such as an automated feedback control loop. A feedback control loop may be applied to any of the fluid treatment systems provided herein, including, but not limited to, topside applications in oil and gas, desalination, cooling water treatment, and boiler water treatment processes, and other water or wastewater treatment processes (e.g., scale inhibition in wastewater, biogas, etc.).

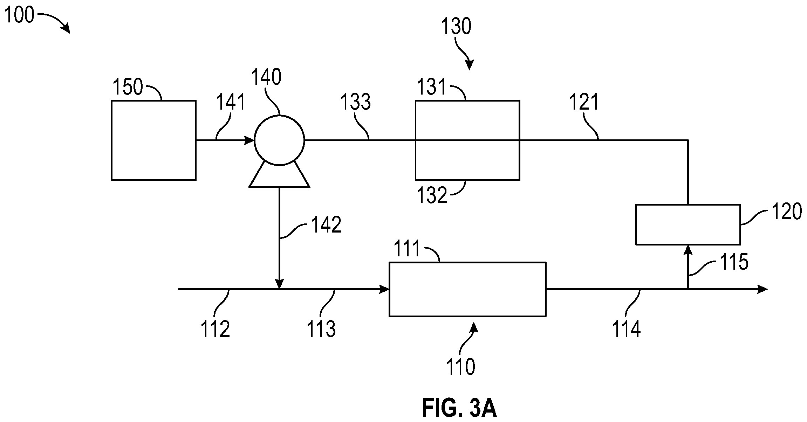

A schematic of an embodiment of a feedback control loop system is depicted at **FIG. 3A****.** The system **100** of **FIG. 3A** includes a fluid treatment system **110** having a fluid treatment apparatus **111,** a fluid stream **112,** an inlet stream **113,** and an outlet stream **114.** The system **100** includes an analyzer including a fluorometer **120** that is in fluid communication with the outlet stream **114** via a side stream **115** drawn from the outlet stream **114.** The system **100** also includes a controller **130** having a processor **131** and a memory unit **132,** which stores a dosing algorithm. The system **100** also includes a pump **140** that is in fluid communication with a reservoir **150** in which an anti-scalant composition is disposed. The pump **140** also is in fluid communication with the fluid stream **112** of the fluid treatment system **110.** The reservoir **150** provides a stream **141** including the anti-scalant composition, and the pump **140** is configured to add an amount **142** of the anti-scalant composition to the fluid stream **112** to generate the inlet stream **113** for the fluid treatment apparatus **111** of the fluid treatment system **110.** The processor **131** is configured to receive from the analyzer including a fluorometer **120** a first signal **121** including fluorometry data collected by the analyzer including a fluorometer **120** from the outlet stream **114** via the side stream **115.** The processor **131** also is configured to determine a dosing instruction by applying the fluorometry data to the dosing algorithm stored by the memory unit **132,** and provide a second signal **133** including the dosing instruction to the pump **140.** The pump **140** is configured to modify the amount of the anti-scalant composition **142** that is added to the fluid stream **112** based on the dosing instruction. The first signal **121** may be transmitted via a physical connection (e.g., a wire) or wirelessly. The second signal **133** may be transmitted via a physical connection (e.g., a wire) or wirelessly.

In some embodiments, the systems or methods herein include a feedforward control loop, such as an automated feedforward control loop. A feedforward control loop may be applied to any of the fluid treatment systems herein, including, but not limited to, topside applications in oil and gas, desalination, cooling water treatment, boiling water treatment, and other water or wastewater treatment processes (e.g., scale inhibition in wastewater, biogas, etc.).

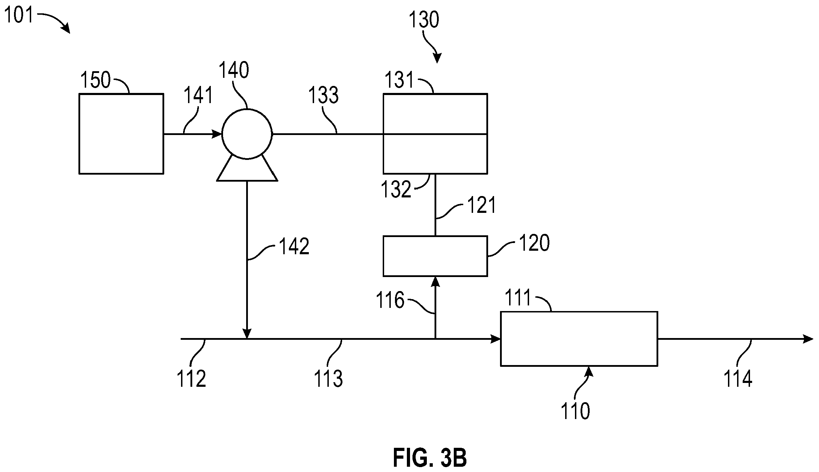

A schematic of an embodiment of a feedback control loop system is depicted at **FIG. 3B****.** The system **101** of **FIG. 3B** includes a fluid treatment system **110** having a fluid treatment apparatus **111,** a fluid stream **112,** an inlet stream **113,** and an outlet stream **114.** The system **101** includes an analyzer including a fluorometer **120** that is in fluid communication with the inlet stream **113** via a side stream **116** drawn from the inlet stream **113.** The system **101** also includes a controller **130** having a processor **131** and a memory unit **132,** which stores a dosing algorithm. The system **101** also includes a pump **140** that is in fluid communication with a reservoir **150** in which an anti-scalant composition is disposed. The pump **140** also is in fluid communication with the fluid stream **112** of the fluid treatment system **110.** The reservoir **150** provides a stream **141** including the anti-scalant composition, and the pump **140** is configured to add an amount **142** of the anti-scalant composition to the fluid stream **112** to generate the inlet stream **113** for the fluid treatment apparatus **111** of the fluid treatment system **110.** The processor **131** is configured to receive from the analyzer including a fluorometer **120** a first signal **121** including fluorometry data collected by the analyzer including a fluorometer **120** from the inlet stream **113** via the side stream **116.** The processor **131** also is configured to determine a dosing instruction by applying the fluorometry data to the dosing algorithm stored by the memory unit **132,** and provide a second signal **133** including the dosing instruction to the pump **140.** The pump **140** is configured to modify the amount of the anti-scalant composition **142** that is added to the fluid stream **112** based on the dosing instruction. The first signal **121** may be transmitted via a physical connection (e.g., a wire) or wirelessly. The second signal **133** may be transmitted via a physical connection (e.g., a wire) or wirelessly.

### Methods

Also provided herein are methods for monitoring and/or controlling dosing of a composition, such as an anti-scalant composition. The methods provided herein may be performed with embodiments of the systems provided herein.

In some embodiments, the methods include providing a system as described herein; adding with the pump an amount of the anti-scalant composition to the fluid stream to create the inlet stream; analyzing the inlet stream and/or the outlet stream with the fluorometer to generate fluorometry data including a first intensity at a fluorescence emission maximum of the fluorescent tracer or the monomer tag at a wavelength pre-selected for the fluorescent tracer or the monomer tag, respectively; transferring a first signal comprising the fluorometry data to the processor of the controller; generating with the controller a first dosing instruction by applying the fluorometry data to the dosing algorithm; transferring a second signal comprising the first dosing instruction to the pump; and modifying with the pump, based on the first dosing instruction, the amount of the anti-scalant composition that is added to the fluid stream so that a first modified amount of the anti-scalant composition is added to the fluid stream.

In some embodiments, the methods include providing a system that includes (i) a fluid treatment system comprising (a) a fluid stream comprising a fluid, (b) an inlet stream, and (c) an outlet stream, (ii) a reservoir in which an anti-scalant composition is disposed, wherein the anti-scalant composition comprises (a) a fluorescent tracer, (b) a tagged polymer comprising a tagging monomer, or (c) a combination thereof, (iii) a pump in fluid communication with (a) the reservoir and (b) the fluid stream of the fluid treatment system, (iv) an analyzer comprising a fluorometer, wherein the fluorometer is in fluid communication with the inlet stream and/or the outlet stream of the fluid treatment system, (v) a controller comprising a processor and a memory unit, and (vi) a dosing algorithm stored by the memory unit of the controller; adding with the pump an amount of the anti-scalant composition to the fluid stream to create the inlet stream; analyzing the inlet stream and/or the outlet stream with the fluorometer to generate fluorometry data comprising a first intensity at a fluorescence emission maximum of the fluorescent tracer or the monomer tag at a wavelength pre-selected for the fluorescent tracer or the monomer tag, respectively; transferring a first signal comprising the fluorometry data to the processor of the controller; generating with the controller a first dosing instruction by applying the fluorometry data to the dosing algorithm; transferring a second signal comprising the first dosing instruction to the pump; and modifying with the pump, based on the first dosing instruction, the amount of the anti-scalant composition that is added to the fluid stream so that a first modified amount of the anti-scalant composition is added to the fluid stream.

In some embodiments, the methods also include analyzing the inlet stream and/or the outlet stream with the fluorometer to generate additional fluorometry data that include a second intensity at the fluorescence emission maximum of the fluorescent tracer or the monomer tag at a wavelength pre-selected for the fluorescent tracer or the monomer tag, respectively; transferring a third signal comprising the additional fluorometry data to the processor of the controller; generating with the controller a second dosing instruction by applying the additional fluorometry data to the dosing algorithm; transferring a fourth signal comprising the second dosing instruction to the pump; and modifying with the pump, based on the second dosing instruction, the first modified amount of the anti-scalant composition that is added to the fluid stream so that a second modified amount of the anti-scalant composition is added to the fluid stream.

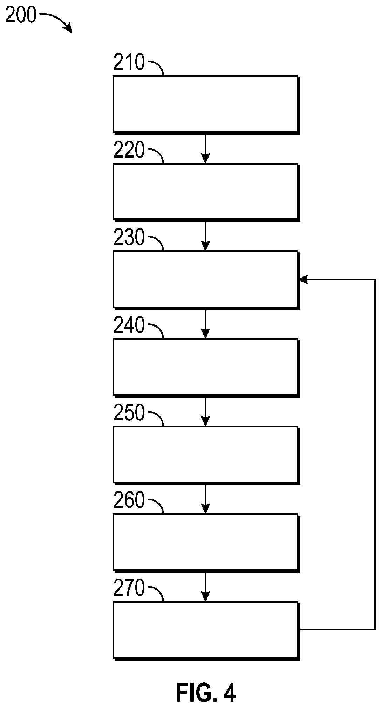

**FIG. 4** is a flow chart depicting elements of an embodiment of a method. The method **200** of **FIG. 4** includes providing **210** a system described herein, wherein the system includes a fluid treatment system having a fluid stream. The method **200** includes adding **220** with the pump an amount of the anti-scalant composition to the fluid stream to create the inlet stream. The method **200** includes analyzing **230** an inlet stream and/or an outlet stream of the fluid treatment system with a fluorometer to generate fluorometry data. The method **200** includes transferring **240** a first signal comprising the fluorometry data to the processor of the controller. The method **200** includes generating **250** with the controller a first dosing instruction by applying the fluorometry data to the dosing algorithm. The method **200** includes transferring **260** a second signal that includes the first dosing instruction to the pump. The method includes modifying **270** with the pump, based on the first dosing instruction, the amount of the anti-scalant composition that is added to the fluid stream so that a first modified amount of the anti-scalant composition is added to the fluid stream. The method **200,** optionally, may include repeating one or more times the analyzing **230,** the transferring **240** of the first signal, the generating **250,** the transferring **260** of the second signal, and the modifying **270,** wherein, independently, each of the analyzing **230,** the transferring **240** of the first signal, the generating **250,** the transferring **260** of the second signal, and the modifying **270** is repeated continuously or intermittently.

In some embodiments, an inlet stream and/or outlet stream is subjected to a pretreatment process prior to being analyzed by a fluorometer. In some embodiments, the pretreatment process includes performing HPLC on the inlet stream or the outlet stream. The HPLC/fluorometry analysis may permit a fluorescent tracer to be separated from a fluid, such as a fluid of an inlet stream or an outlet stream, prior to collecting fluorometry data. In some embodiments, the pretreatment process includes contacting the inlet stream or the outlet stream with molecular sieves, adjusting the pH of the inlet stream or the outlet stream, or a combination thereof. The adjusting of the pH may include reducing a pH of a stream to an acidic pH (e.g., about 1) or increasing the pH to a basic pH (e.g., about 11).

For example, when a quinicine tagged anti-scalant composition is used in the systems or methods provided herein, the methods may include contacting the inlet and/or outlet stream with molecular sieves, which may remove chloride ions, and adjusting the pH of the stream to about 1 with an acid, such as sulfuric acid. Following this pretreatment, the quinicine tagged anti-scalant composition may be analyzed with a fluorometer according to the following parameters: excitation 260 nm, emission max 440 nm (emission could be collected from an emission range of about 300 nm to about 600 nm). As a further example, when sodium styrene sulfonate tagged polymers are used as an anti-scalant composition, then a wider pH range at measurement may be acceptable, thereby permitting a pH adjustment to be avoided. Therefore, when sodium styrene sulfonate tagged polymers are used as an anti-scalant composition in the systems or methods provided herein, the methods may include contacting the inlet stream and/or the outlet stream with molecular sieves, and then analyzing the inlet stream and/or the outlet stream with a fluorometer according to the following parameters: excitation 225 nm, emission max 290 nm.

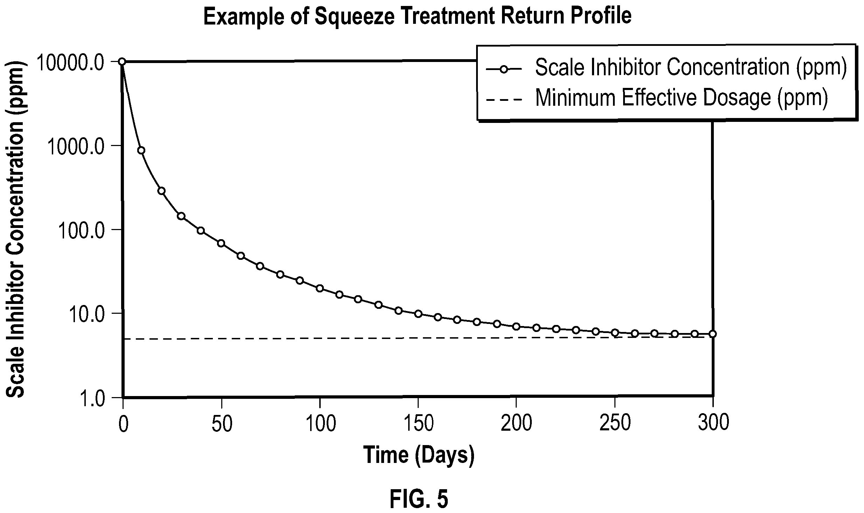

In some embodiments, the methods include monitoring, but not controlling, a concentration of an anti-scale composition. For example, the systems or methods herein may be used to generate a squeeze treatment return profile. An example of a squeeze treatment return profile is depicted at **FIG. 5****.** When the systems or methods herein are used to generate a squeeze treatment return profile, fluorometry data may be collected intermittently, such as once per day. When a concentration of an anti-scalant composition is below a minimum effective dosage, a new squeeze treatment is required. Monitoring a concentration of an anti-scalant composition online with the systems or methods provided herein may provide a more accurate measurement at least in part because a sample is not exposed to air, the measurement is performed at fluid conditions (e.g., pressure, temperature, etc.), or a combination thereof, thereby minimizing undesirable changes in the samples. After a squeeze treatment, the concentration of anti-scale composition usually is relatively high (for example, 10,000 ppm to 50,000 ppm, which, without sample dilution, is too high for performing a fluorescent measurement). The concentration of anti-scalant composition, however, typically decreases within days to about 100 ppm to about 1,000 ppm, and then continues to decrease slowly over time.

In some embodiments, the adding of (i) the amount of the anti-scalant composition to the fluid stream, (ii) the first modified amount of the anti-scalant composition, (iii) the second modified amount of the anti-scalant composition, or (iv) a combination thereof is performed continuously.

In some embodiments, the adding of (i) the amount of the anti-scalant composition to the fluid stream, (ii) the first modified amount of the anti-scalant composition, (iii) the second modified amount of the anti-scalant composition, or (iv) a combination thereof is performed intermittently.

In some embodiments, the generating of (i) the fluorometry data, (ii) the additional fluorometry data, or (iii) a combination thereof is performed intermittently.

In some embodiments, (i) the amount of the anti-scalant composition that is added to the fluid stream is less than the first modified amount of the anti-scalant composition that is added to the fluid stream, (ii) the amount of the anti-scalant composition that is added to the fluid stream is greater than the first modified amount of the anti-scalant composition that is added to the fluid stream, or (iii) the amount of the anti-scalant composition that is added to the fluid stream is equal to the first modified amount of the anti-scalant composition that is added to the fluid stream.

In some embodiments, (i) the first modified amount of the anti-scalant composition that is added to the fluid stream is less than the second modified amount of the anti-scalant composition that is added to the fluid stream, (ii) the first modified amount of the anti-scalant composition that is added to the fluid stream is greater than the second modified amount of the anti-scalant composition that is added to the fluid stream, or (iii) the first modified amount of the anti-scalant composition that is added to the fluid stream is equal to the second modified amount of the anti-scalant composition that is added to the fluid stream.

Generally, (i) the amount of the anti-scalant composition, (ii) the first modified amount of the anti-scalant composition, (iii) the second modified amount of the anti-scalant composition, or (iv) a combination thereof may include any amount of anti-scalant composition that produces and/or maintains a necessary or desired effect in a fluid treatment system, such as an amount that prevents or reduces scale formation.

The effective scale-inhibiting amount of a scale-inhibiting composition may generally depend on a particular system to be treated and scale-inhibiting moieties in the scale-inhibiting composition. For example, the effective scale-inhibiting amount of an anti-scale composition in a particular system to be treated may be influenced by factors such as the area subject to deposition, pH, temperature, water quality, the respective concentration in the water of the potential scale and deposit forming species, or a combination thereof.

In some embodiments, an anti-scale composition is effective in a system to be treated when the scale-inhibiting composition is provided at levels less than about 200 parts per million (ppm), less than about 100 ppm, less than about 50 ppm, less than about 35 ppm, less than about 20 ppm, less than about 10 ppm, or less than 1 ppm on the basis of the fluid in a system to be treated. In some embodiments, the anti-scale composition is effective at concentrations of about 0.01 ppm to about 200 ppm, 0.5 ppm to about 200 ppm, about 0.01 ppm to about 100 ppm, about 0.5 ppm to about 100 ppm, about 0.5 ppm to about 50 ppm, about 0.5 ppm to about 35 ppm, about 0.5 ppm to about 10 parts ppm, about 0.5 ppm to about 3 parts ppm, about 2 ppm to about 10 ppm, or about 4 ppm to about 7 ppm. In some embodiments, the methods and systems provided herein are used in desalination applications, and an anti-scale composition is provided at a concentration of about 0.01 ppm to about 20 ppm. In some embodiments, the methods and systems provided herein are used in oil topside applications, and an anti-scale composition is provided at a concentration of about 10 ppm to about 50 ppm.

In some embodiments, an effective scale-inhibiting amount is an amount sufficient to inhibit calcium carbonate, calcium sulfate, barium sulfate, strontium sulfate, halite, iron sulfide, magnesium carbonate, barium carbonate, strontium carbonate, calcium fluoride, magnesium hydroxide, silica, silicate scales, lead sulfide, and/or calcium phosphate scale formation; and/or other carbonate, sulfate, and/or phosphate containing scales.

### Anti-scalant Compositions

Generally, any anti-scalant composition that includes a fluorescent tracer, a tagging monomer, or a combination thereof may be used in the systems and methods provided herein. A tagging monomer (or "monomer tag" or the like) is a fluorescent tracer that is a monomer of a polymer chain (e.g., a polymer backbone).

A fluorescent tracer may be dispersed in one or more components of an anti-scalant composition, covalently bonded to one or more components of an anti-scalant composition, ionically bonded to one or more components of an anti-scalant composition, or a combination thereof. A tagging monomer is a compound that may be polymerized alone, or with one or more other monomers to produce a polymeric anti-scalant composition.

The term "anti-scalant", the phrases "scale inhibition", "scale inhibitor" or "scale-inhibiting", and the like generally refer to materials (e.g., compounds, monomers, polymer compositions, etc.) that may be applied (e.g., at substoichiometric levels) to interfere with crystal nucleation, growth, agglomeration, or a combination thereof. As used herein, the terms "anti-scalant", the phrases "anti-scale agent" and "scale inhibitor", and the like are used in their ordinary sense as understood by one skilled in the art, and thus may be used herein to refer to or describe chemical compounds or compositions, such as polymer compositions, containing such compounds, where the compounds, when added to an system, reduce or inhibit the amount of scale and/or rate of formation of scale in the system, as compared to a system that does not contain the added chemical compound or composition. In this context, the term "scale" or the phrase "mineral scale" refer to insoluble substances, such as insoluble salts, that may have a tendency to form in aqueous systems, such as boiler water, cooling water, seawater (e.g. in oil platform applications), brackish water, oilfield water, municipal treatment plant water, paper mill water, mining water, industrial treatment plant water, etc.

The phrases "treatment of scale", "treated for scale", "preventing or reducing scale formation", and the like will be understood by those skilled in the art to have a broad and customary meaning that includes using the scale-inhibiting polymer compositions herein to (i) reduce an amount of scale, (ii) inhibit an amount of scale, (iii) reduce a rate of formation of scale, or (iv) a combination thereof in various systems, including aqueous systems, as compared to comparable systems that do not contain the anti-scale polymer composition.

### Tagging Monomers

Tagging monomers are provided herein, which may be used as fluorescent tagging monomers in polymers, including those disclosed herein. As used herein, the phrases "tagging agent", "tagging monomer", and the like refer to a monomer of a polymer that is detectable at a desirable concentration (e.g., a relatively low concentration) using an analytical technique, such as fluorescence spectroscopy.

The tagging monomers provided herein, in some embodiments, exhibit a fluorescence emission maximum at about 410 nm to about 680 nm, about 410 nm to about 600 nm, about 410 nm to about 590 nm, about 410 nm to about 520 nm, about 410 nm to about 500 nm, about 440 nm to about 450 nm, about 500 nm to about 520 nm, about 550 nm to about 590 nm, about 640 nm to about 680 nm, or about 570 nm to about 600 nm, thereby providing polymer compositions or other products with a feature that may permit an amount (e.g., a concentration) of a polymer composition that includes a tagging monomer to be monitored. When, for example, an anti-scalant composition includes (i) sodium styrene sulfonate, the emission maximum may be at 290 nm, or (ii) quinicine, the emission maximum may be at 440 nm. When two or more fluorescent tracers and/or tagged monomers are present in an anti-scalant composition, each fluorescent tracer and/or tagged monomer may exhibit a different fluorescence emission. For example, an anti-scalant composition may include (i) a first copolymer including a first monomer, such as resorcinmalein, which has a fluorescence emission maximum of about 500 to about 520 nm, (ii) a second copolymer including a first monomer, such as one based on diethylaminophenol, which has a fluorescence emission maximum of about 550 nm to about 590 nm, (iii) a third copolymer including a first monomer, such as naphthomalein, which has a fluorescence emission maximum of about 640 nm to about 680 nm, or (iv) a combination thereof. In some embodiments, the excitation and emission wavelengths are determined, and, therefore, may be adjusted, by selecting a particular arylalcohol/amino aryl alcohol. The fluorescence emission may be affected by pH; for example, the keto-enol tautomers described herein may exhibit different fluorescence emissions.

Generally, the tagging monomer may include any compound that is polymerizable and detectable at a desirable concentration (e.g., a relatively low concentration) using an analytical technique, such as fluorescence spectroscopy. In some embodiments, the tagging monomer includes sodium styrene sulfonate.

The compounds, including tagging monomers, provided herein include compounds or isomers of Formula (I), Formula (II), Formula (III), or Formula (IV). The phrases "compound of Formula (I)", "compound of Formula (II)", the term "compound" when it refers to Formula (I), (II), (III), or (IV), the term "isomer" when it refers to Formula (III) or Formula (IV), and the like, as used herein, refer to and include compounds according to or isomers of, respectively, the structures of each formula, salts thereof, hydrates thereof, salt hydrates thereof, stereoisomers thereof, dehydrates thereof, and derivatives thereof. Therefore, the formulas and structures provided herein encompass and read on the formulas and structures as drawn or isomers of the formulas and structures as drawn, as well as salts, hydrates, salt hydrates, stereoisomers, dehydrates, tautomers, or derivatives of each formula and structure or isomer thereof. The "derivatives" of each formula and structure include, but are not limited, to polymers (e.g., oligomers, copolymers, etc.) formed of the compounds. The tautomers may include keto-enol tautomers.

The compounds provided herein include compounds or isomers of Formula (I), Formula (II), Formula (III), and Formula (IV), which, as explained herein, include salts, hydrates, salt hydrates, stereoisomers, dehydrates, tautomers, and derivatives of the compounds or isomers of Formula (I), Formula (II), Formula (III), and Formula (IV): 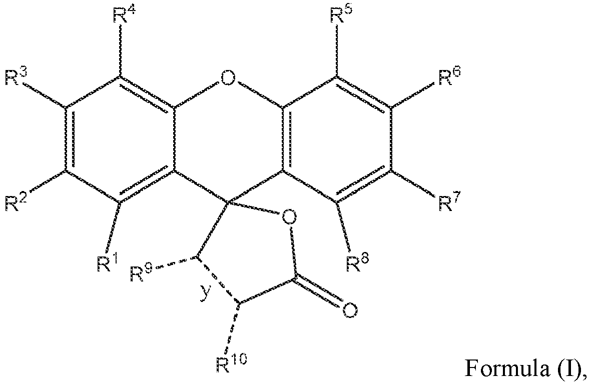 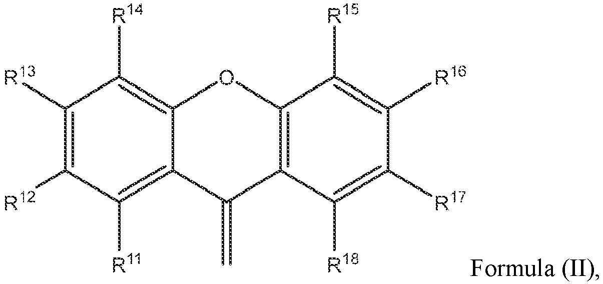 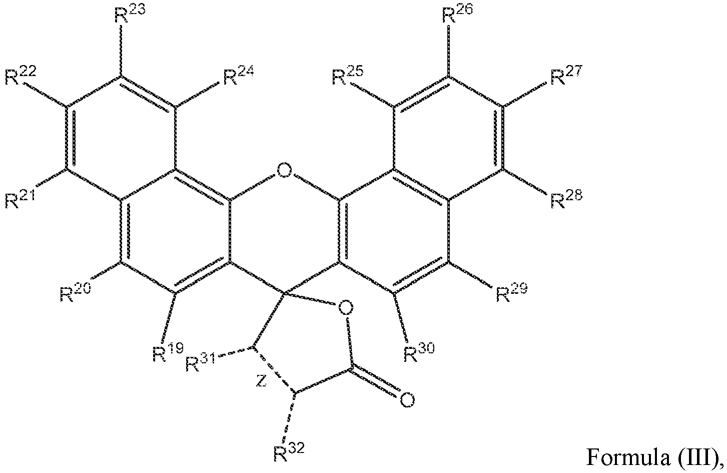 or 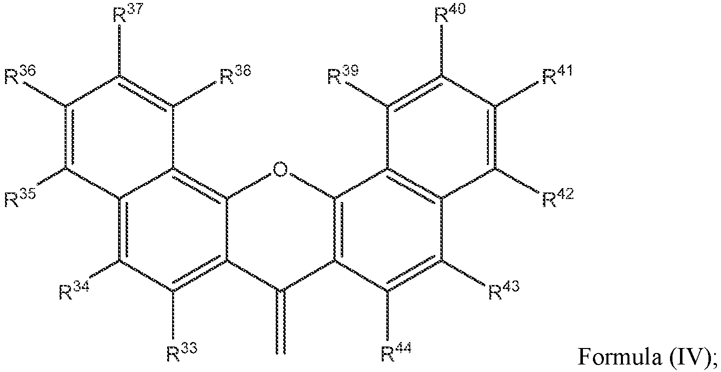 wherein R¹, R², R³, R⁴, R⁵, R⁶, R⁷, R⁸, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹, R³⁰, R³³, R³⁴, R³⁵, R³⁶, R³⁷, R³⁸, R³⁹, R⁴⁰, R⁴¹, R⁴², R⁴³, and R⁴⁴ are independently selected from the group consisting of hydrogen, hydroxyl, C₁-C₆ alkoxy, C₂-C₆ alkenoxy, C₂-C₆ alkynoxy, -N(R')(R"), C₁-C₆ alkyl, C₂-C₆ alkenyl, C₂-C₆ alkynyl, and C₄-C₁₄ aryl; wherein R' and R" are independently selected from the group consisting of hydrogen and C₁-C₆ alkyl; wherein R⁹, R¹⁰, R³¹, and R³² are independently selected from the group consisting of hydrogen, hydroxyl, C₁-C₆ alkyl, and C₁-C₆ alkenyl; wherein y is a single bond or a double bond; wherein z is a single bond or a double bond;
wherein the isomers of Formula (III) include the following isomers - 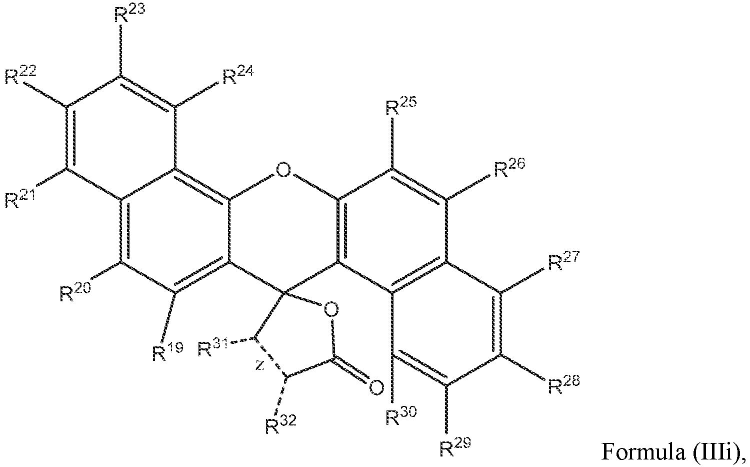 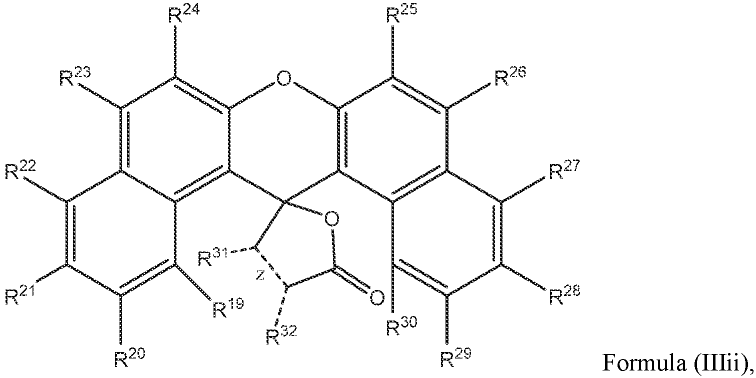 and/or 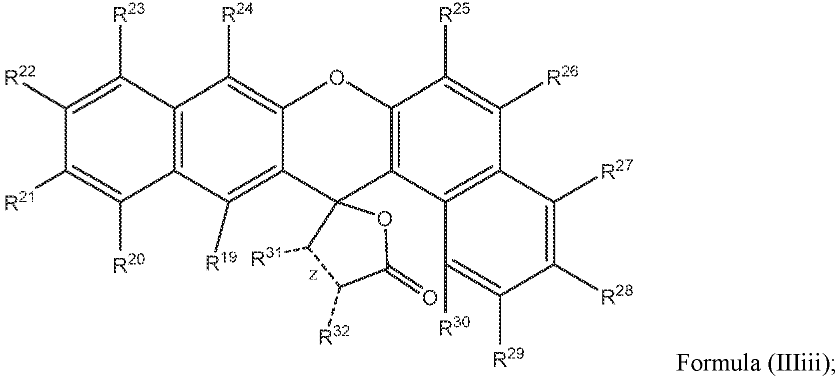 and wherein the isomers of Formula (IV) include the following isomers - 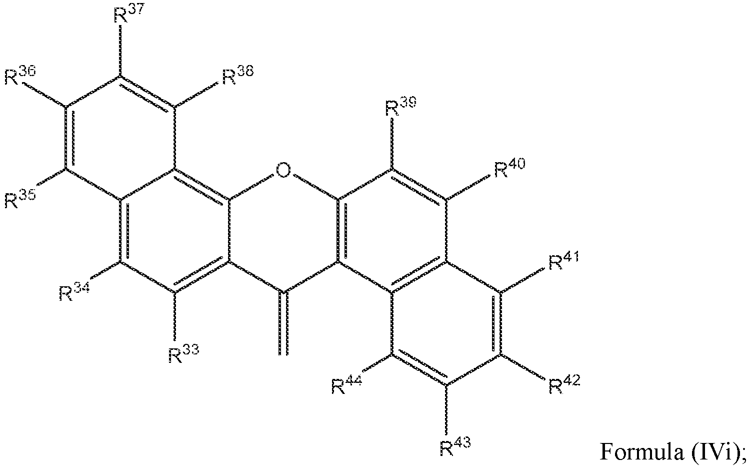 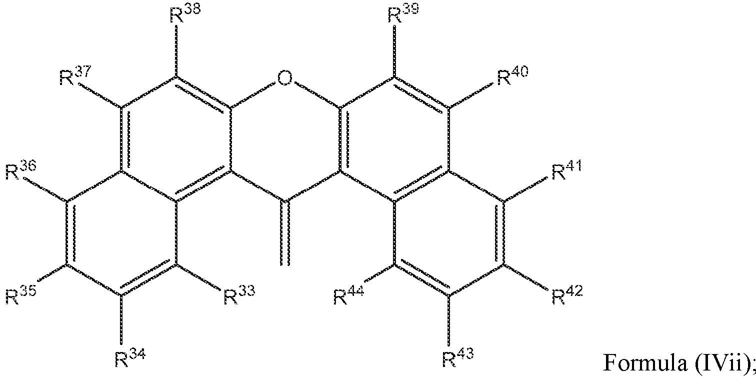 and/or

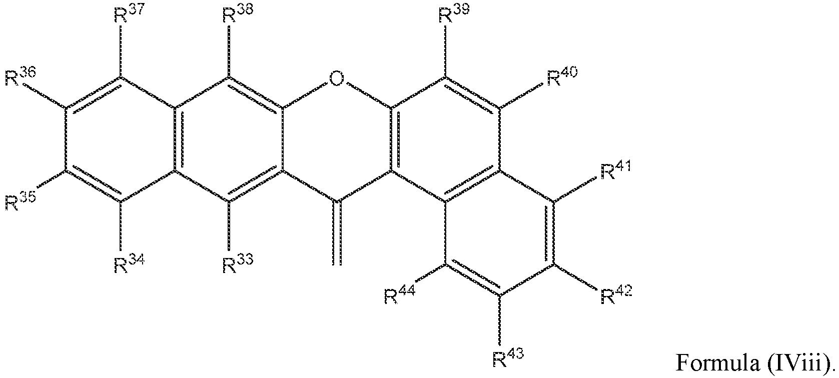

In some embodiments, (i) y is a double bond or (ii) R⁹ is a C₁ alkenyl. In some embodiments, (i) z is a double bond or (ii) R³¹ is a C₁ alkenyl.

The compounds provided herein may include "R" groups (e.g., R¹, R², etc.) selected from C₁-C₆ alkoxy, C₂-C₆ alkenoxy, C₂-C₆ alkynoxy, C₁-C₆ alkyl, C₂-C₆ alkenyl, C₂-C₆ alkynyl, C₄-C₁₄ aryl, C₁-C₆ alkenyl, and the like.

Each C₁-C₆ alkoxy, C₂-C₆ alkenoxy, C₂-C₆ alkynoxy, C₁-C₆ alkyl, C₂-C₆ alkenyl, C₂-C₆ alkynyl, C₄-C₁₄ aryl, C₁-C₆ alkenyl, and the like disclosed herein, includes all substituted, unsubstituted, branched, and linear analogs or derivatives thereof, in each instance having the indicated number of carbon atoms. Examples of alkyl groups include, but are not limited to, methyl, ethyl, propyl, isopropyl, n-butyl, t-butyl, isobutyl, pentyl, hexyl, isohexyl, cyclopropyl, cyclobutyl, cyclopentyl, and cyclohexyl. Additional examples of alkyl moieties have linear, branched and/or cyclic portions. Representative alkenyl moieties include, but are not limited to, vinyl, allyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and 1-hexenyl. Representative alkynyl moieties include, but are not limited to, acetylenyl, propynyl, 1-butynyl, 2-butynyl, 1-pentynyl, 2-pentynyl, 3-methyl-1-butynyl, 4-pentynyl, 1-hexynyl, 2-hexynyl, and 5-hexynyl. Examples of alkoxy, alkenoxy, and alkyoxy compounds include any of the foregoing alkyl groups, alkenyl groups, or aklynyl groups that are covalently bonded to an oxygen atom. Examples of aryl moieties include, but are not limited to, anthracenyl, azulenyl, biphenyl, fluorenyl, indan, indenyl, naphthyl, phenyl, 1,2,3,4-tetrahydro-naphthalene, and the like, including substituted derivatives thereof, in each instance having from 4 to about 14 carbons. Substituted derivatives of aromatic compounds include, but are not limited to, tolyl, xylyl, mesityl, and the like, including any heteroatom substituted derivative thereof.

In the structures provided herein, such as Formula (I) and Formula (III), chemical bonds represented by a dotted line may be a double bond or a single bond. For example, as indicated herein, "y" and "z" may independently be a single bond or double bond. Also, for example, the covalent bond between R⁹ and the lactone moiety of Formula (I) may be a single bond or double bond, depending on the selection made for R⁹. When, for example, R⁹ is an unsubstituted C₁ alkenyl, then R⁹ is double bonded to the lactone moiety, y is a single bond, and R¹⁰ is single bonded to the lactone moiety:

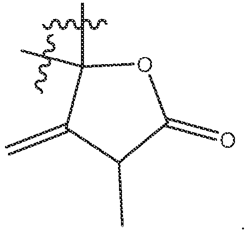

As a further example, the covalent bond between R¹⁰ and the lactone moiety of Formula (I) may be a single bond or double bond, depending on the selection made for R¹⁰. When, for example, R¹⁰ is an unsubstituted C₁ alkenyl, then R¹⁰ is double bonded to the lactone moiety, y is a single bond, and R¹⁰ (e.g., a hydrogen as shown below) is single bonded to the lactone moiety:

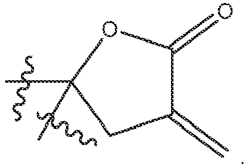

Each C₄-C₁₄ aryl group of the compounds provided herein may independently include (i) a single "R" substituent (for example, one of R¹, R², R³, R⁴, R⁷, R⁸, R⁹, or R¹⁰), or (ii) at least two "R" substituents on adjacent carbon atoms (for example, R¹ and R², wherein R¹ and R² are covalently bonded to each other; R² and R³, wherein R² and R³ are covalently bonded to each other; R³ and R⁴, wherein R³ and R⁴ are covalently bonded to each other; etc.). Therefore, for example, in Formula (I), an unsubstituted C₆ aryl group (i.e., a phenyl) may be selected for each of R² and R³ (Structure (a)), or an unsubstituted C₄ aryl group may be selected jointly for R² and R³, thereby resulting in a 6-membered aryl ring that includes the carbon atom to which R² is covalently bonded, the carbon atom to which R³ is covalently bonded, R², and R³, wherein R² and R³ are covalently bonded to each other (Structure (b)):

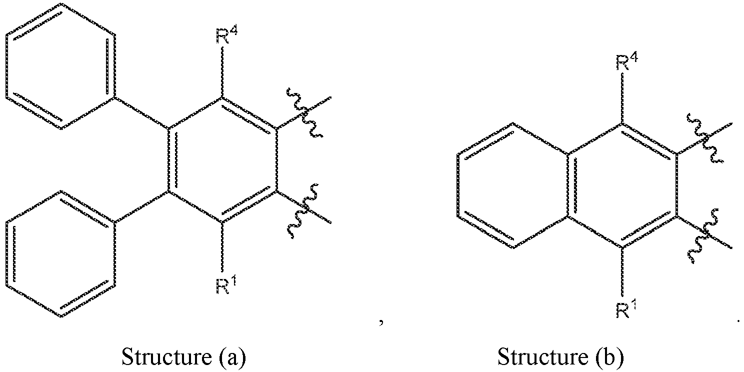

Each C₁-C₆ alkoxy, C₂-C₆ alkenoxy, C₁-C₆ alkyl, C₂-C₆ alkenyl, and/or C₁-C₆ alkenyl of the compounds provided herein may independently include (i) a single "R" substituent (for example, one of R', R", R¹, R², R³, R⁴, R⁷, R⁸, R⁹, or R¹⁰), or (ii) at least two "R" substituents on adjacent carbon atoms (for example, R¹ and R², wherein R¹ and R² are covalently bonded to each other; R² and R³, wherein R² and R³ are covalently bonded to each other; R³ and R⁴, wherein R³ and R⁴ are covalently bonded to each other; R' and R², wherein R' and R² are covalently bonded to each other, etc.). Therefore, for example, in Formula (I), an unsubstituted C₆ alkyl group (i.e., a hexyl) may be selected for each of R² and R³ (Structure (a)), or an unsubstituted C₄ alkyl group may be selected jointly for R² and R³, thereby resulting in a 6-membered ring that includes the carbon atom to which R² is covalently bonded, the carbon atom to which R³ is covalently bonded, R², and R³, wherein R² and R³ are covalently bonded to each other (Structure (b)):

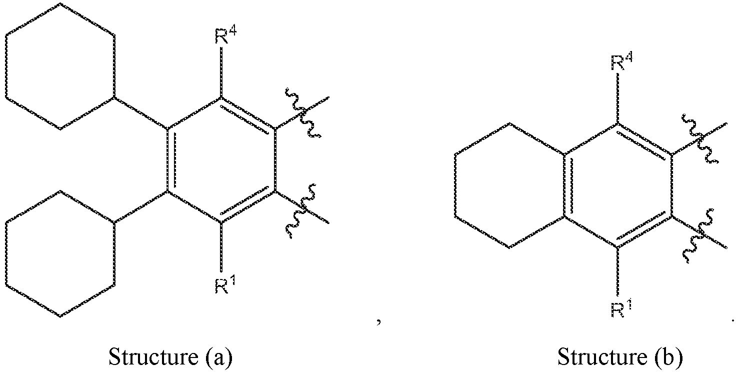

Unless otherwise indicated, the term "substituted," when used to describe a chemical structure or moiety, refers to a derivative of that structure or moiety wherein one or more of its hydrogen atoms is substituted with a chemical moiety or functional group such as alcohol, alkoxy, alkanoyloxy, alkoxycarbonyl, alkenyl, alkyl (e.g., methyl, ethyl, propyl, t-butyl), alkynyl, alkylcarbonyloxy (-OC(O)alkyl), amide (-C(O)NH-alkyl- or -alkylNHC(O)alkyl), tertiary amine (such as alkylamino, arylamino, arylalkylamino), aryl, aryloxy, azo, carbamoyl (-NHC(O)O-alkyl- or -OC(O)NH-alkyl), carbamyl (e.g., CONH₂, as well as CONH-alkyl, CONH-aryl, and CONH-arylalkyl), carboxyl, carboxylic acid, cyano, ester, ether (e.g., methoxy, ethoxy), halo, haloalkyl (e.g., -CCl₃, -CF₃, -C(CF₃)₃), heteroalkyl, isocyanate, isothiocyanate, nitrile, nitro, phosphodiester, sulfide, sulfonamido (e.g., SO₂NH₂), sulfone, sulfonyl (including alkylsulfonyl, arylsulfonyl and arylalkylsulfonyl), sulfoxide, thiol (e.g., sulfhydryl, thioether), urea (-NHCONH-alkyl-), or a combination thereof. When an "R" group (e.g., R¹) is substituted, the carbon atoms in the substituents are included in the total count of carbon atoms in the "R" group. For example, if R¹ is selected from a C₁-C₆ alkyl, and the C₁-C₆ alkyl is a propyl group substituted with a dimethylamine substituent, then R¹ is considered, in this example, to be a C₅ alkyl because there are 3 carbon atoms in the propyl group, and 2 carbon atoms in the dimethylamine substituent.

When a compound or isomer of Formula (I), Formula (II), Formula (III), or Formula (IV) includes a stereocenter, the compounds or isomers of Formula (I), Formula (II), Formula (III), or Formula (IV) include both of the (*R*) and (*S*) enantiomers. For example, Formula (IIIiii), as drawn, includes both of the following stereoisomers:

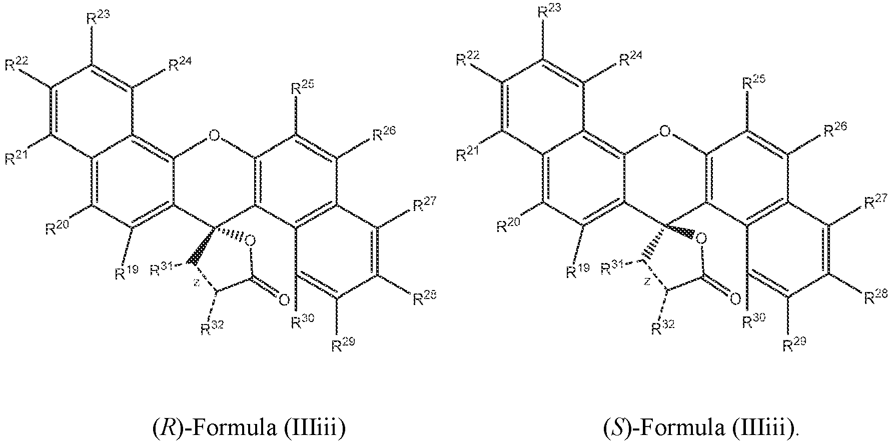

In some embodiments, the compound is a compound of Formula (I), wherein R⁹ is an unsubstituted C₁ alkyl, y is a double bond, and R¹⁰ is hydrogen:

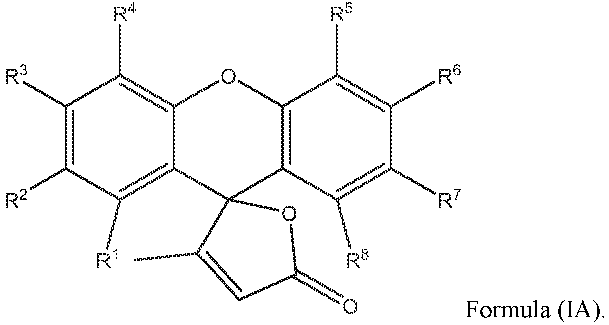

In some embodiments, the compound is a compound of Formula (IA), wherein at least one of R¹, R², R³, and R⁴ is hydroxyl, at least one of R⁵, R⁶, R⁷, and R⁸ is hydroxyl, and any remaining members of R¹-R⁸ are hydrogen:

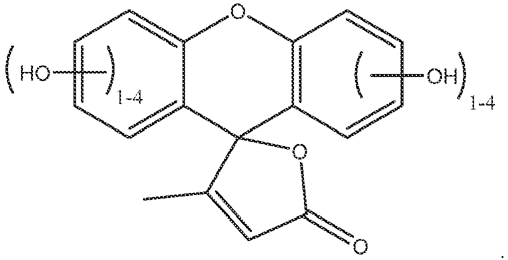

In some embodiments, the compound is a compound of Formula (IA), wherein R¹, R², R⁴, R⁵, R⁷, R⁸, and R¹⁰ are hydrogen, R³ and R⁶ are hydroxyl, and the compound is 3',6'-dihydroxy-3-methyl-5H-spiro[furan-2,9'-xanthen]-5-one, a salt, a hydrate, a salt hydrate, a stereoisomer, a dehydrate, a tautomer, or a derivative thereof -

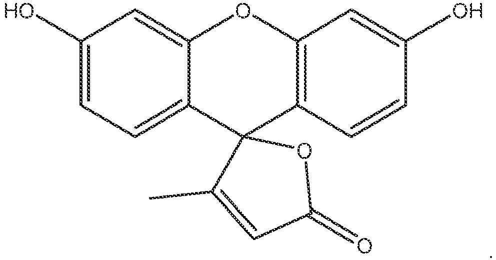

In some embodiments, the compound is a compound of Formula (IA), wherein at least one of R¹, R², R³, and R⁴ is -N(R')(R"), at least one of R⁵, R⁶, R⁷, and R⁸ is -N(R')(R"), and any remaining members of R¹-R⁸ are hydrogen:

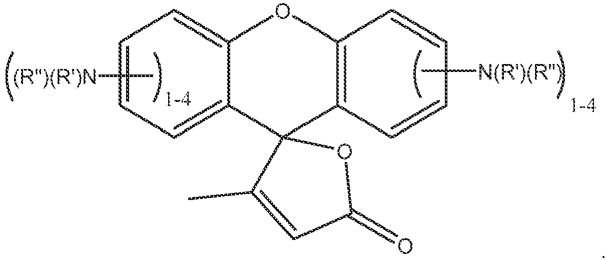

In some embodiments, the compound is a compound of Formula (IA), wherein R¹, R², R⁴, R⁵, R⁷, R⁸, and R¹⁰ are hydrogen, R³ and R⁶ are -N(R')(R"), R' and R" are unsubstituted C₂ alkyl, and the compound is 3',6'-bis(diethylamino)-3-methyl-5*H*-spiro[furan-2,9'-xanthen]-5-one, a salt, a hydrate, a salt hydrate, a stereoisomer, a dehydrate, a tautomer, or a derivative thereof -

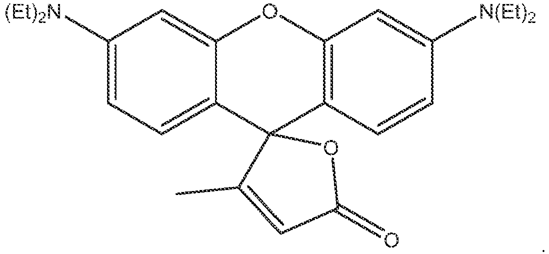

For example, a tautomer of 3',6'-bis(diethylamino)-3-methyl-5*H*-spiro[furan-2,9'-xanthen]-5-one may include (*Z*)-*N*-(9-(1-carboxyprop-1-en-2-yl)-6-(diethylamino)-3*H*-xanthen-3-ylidene)-N-ethylethanaminium, as depicted in the following scheme: 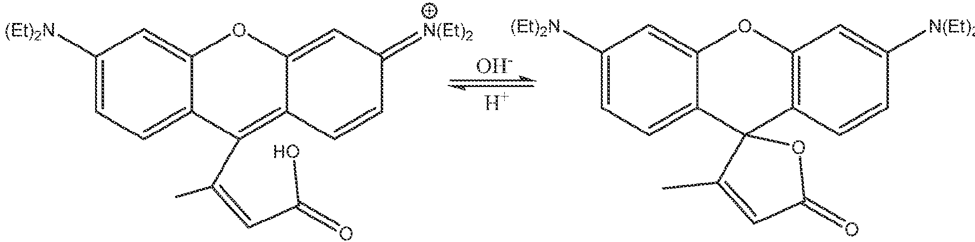 As explained herein, the fluorescence emission of the foregoing tautomers may differ; therefore, the fluorescence emission of the compounds or isomers herein may depend, at least in part, on pH. Selection of a pH may permit fluorescence emission to be tuned.

In some embodiments, the compound is a compound of Formula (IA), wherein R¹ and R⁸ are hydrogen, R³ and R⁶ are -N(R')(R"), R' (of R³) and R², jointly, are an unsubstituted C₃ alkyl, R" (of R³) and R⁴, jointly, are an unsubstituted C₃ alkyl, R' (of R⁶) and R⁵, jointly, are an unsubstituted C₃ alkyl, R" (of R⁶) and R⁷, jointly are an unsubstituted C₃ akyl, and the compound has the following structure, including a salt, a hydrate, a salt hydrate, a stereoisomer, a dehydrate, a tautomer, or a derivative thereof -

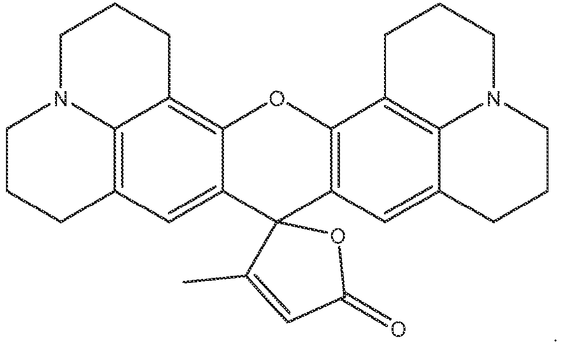

In some embodiments, the compound is a compound of Formula (I), wherein R⁹ is an unsubstituted C₁ alkenyl, y is a single bond, and R¹⁰ is hydrogen:

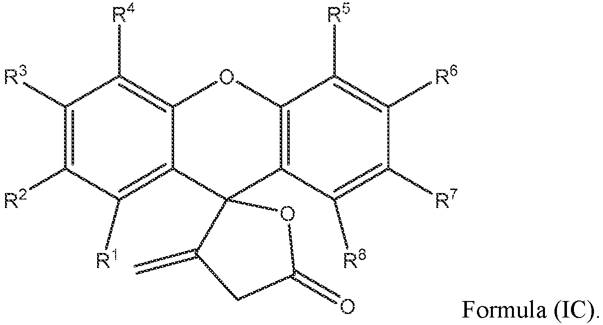

In some embodiments, the compound is a compound of Formula (I), wherein R⁹ and R¹⁰ are unsubstituted C₁ alkyls, and y is a double bond:

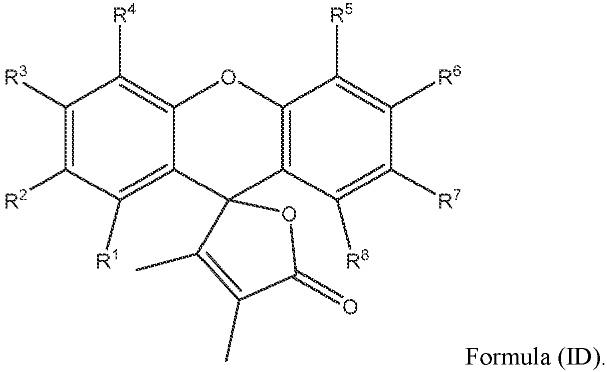

In some embodiments, the compound is a compound of Formula (I), wherein R⁹ and R¹⁰ are hydrogen, and y is a double bond:

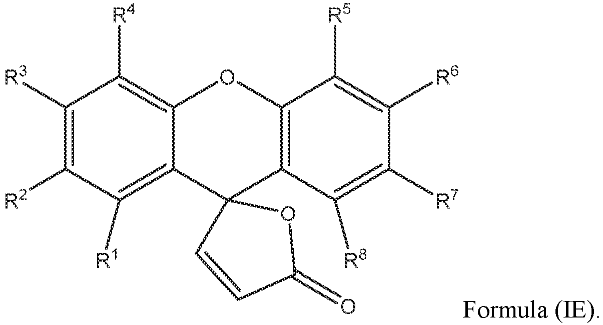

As described herein, the compounds or isomers of Formula (I), Formula (II), Formula (III), or Formula (IV) include tautomers thereof. Therefore, for example, Formula (IE) encompasses and reads on the following tautomers, wherein, in some embodiments, R⁶ of Formula (IE) is a hydroxyl prior to the following tautomerization:

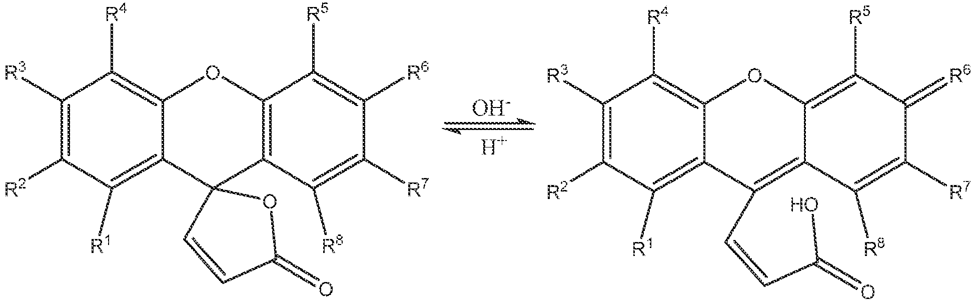

Each of Formulas (IC), (ID), and (IE) may be substituted in the same manner as Formula (IA). For example, in some embodiments, Formula (IE), like Formula (IA), is substituted as follows: R¹ and R⁸ are hydrogen, R³ and R⁶ are -N(R')(R"), R' (of R³) and R², jointly, are a C₃ alkyl, R" (of R³) and R⁴, jointly, are a C₃ alkyl, R' (of R⁶) and R⁵, jointly, are a C₃ alkyl, R" (of R⁶) and R⁷, jointly are a C₃ akyl, and the compound has the following structure, including a salt, a hydrate, a salt hydrate, a stereoisomer, a dehydrate, a tautomer, or a derivative thereof -

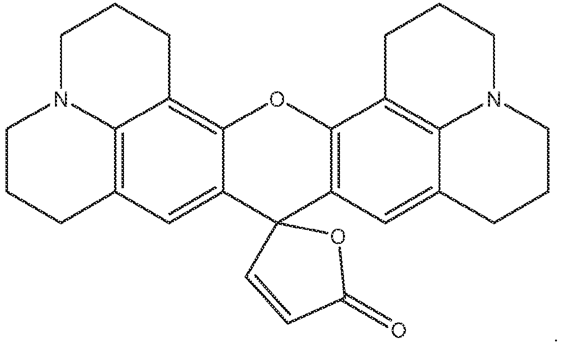

In some embodiments, the compound is a compound of Formula (II), wherein at least one of R¹¹, R¹², R¹³, and R¹⁴ is hydroxyl, at least one of R¹⁵, R¹⁶, R¹⁷, and R¹⁸ is hydroxyl, and the remaining substituents are hydrogen:

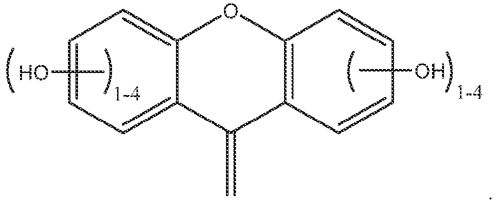

In some embodiments, the compound is a compound of Formula (II), wherein R¹¹, R¹², R¹⁴, R¹⁵, R¹⁷, and R¹⁸ are hydrogen, R¹³ and R¹⁶ are hydroxyl, and the compound is 9-methylene-9H-xanthene-3,6-diol, a salt, a hydrate, a salt hydrate, a stereoisomer, a dehydrate, a tautomer, or a derivative thereof:

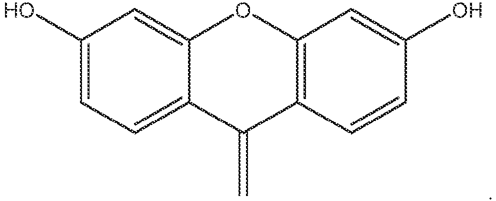

In some embodiments, the compound is a compound of Formula (II), wherein at least one of R¹¹, R¹², R¹³, and R¹⁴ is -N(R')(R"), at least one of R¹⁵, R¹⁶, R¹⁷, and R¹⁸ is -N(R')(R"), and the remaining substituents are hydrogen:

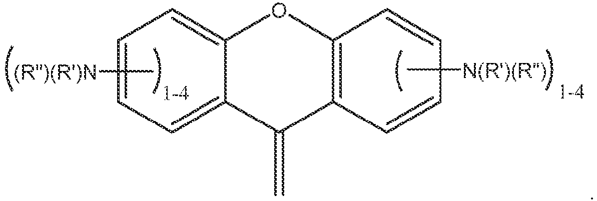

In some embodiments, the compound is a compound of Formula (II), wherein R¹¹, R¹², R¹⁴, R¹⁵, R¹⁷, and R¹⁸ are hydrogen, R¹³ and R¹⁶ are -N(R')(R"), R' and R" are unsubstituted C₂ alkyl, and the compound is *N*³,*N*³,*N*⁶,*N*⁶-tetraethyl-9-methylene-9*H*-xanthene-3,6-diamine, a salt, a hydrate, a salt hydrate, a stereoisomer, a dehydrate, a tautomer, or a derivative thereof:

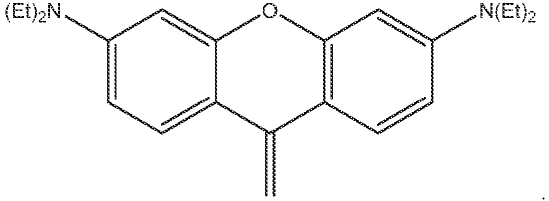

In some embodiments, the compound is a compound or isomer of Formula (III), wherein R³¹ is an unsubstituted C₁ alkyl, z is a double bond, and R³² is hydrogen. When these substituents are selected for Formula (III), the compound is a compound of Formula (IIIA):

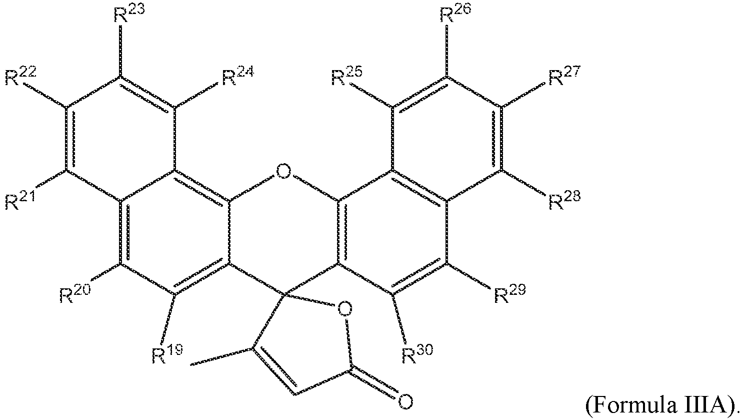

Although Formula (IIIA) is an embodiment of Formula (III), the same substituents may be selected for Formula (IIIi), Formula (IIIii), or Formula (IIIiii), thereby resulting in isomers of Formula (IIIA).

In some embodiments, the compound is a compound or isomer of Formula (IIIA), wherein R¹⁹, R²⁰, R²⁹, and R³⁰ are hydrogen, at least one of R²¹, R²², R²³, and R²⁴ is hydroxyl, at least one of R²⁵, R²⁶, R²⁷, and R²⁸ is hydroxyl, and any remaining members of R²¹-R²⁸ are hydrogen. Such a compound of Formula (IIIA) has the following structure:

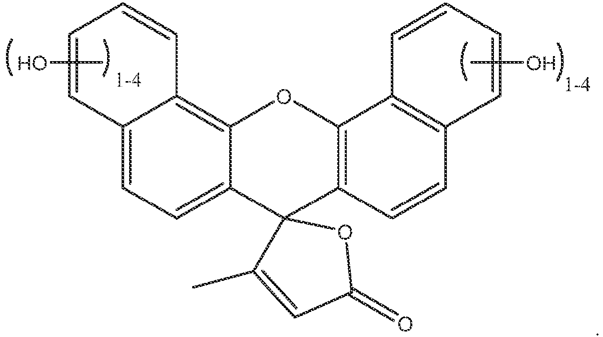

In some embodiments, the compound is a compound or isomer of Formula (IIIA), wherein R¹⁹, R²⁰, R²¹, R²³, R²⁴, R²⁵, R²⁶, R²⁸, R²⁹, and R³⁰ are hydrogen, R²² and R²⁷ are hydroxyl, and the compound is 3,11-dihydroxy-3'-methyl-5'*H*-spiro[dibenzo[c,h]xanthene-7,2'-furan]-5'-one, a salt, a hydrate, a salt hydrate, a stereoisomer, a dehydrate, a tautomer, or a derivative thereof-

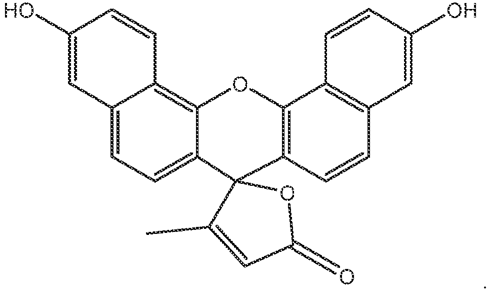

In some embodiments, the compound is a compound or isomer of Formula (IIIA), wherein R¹⁹, R²⁰, R²¹, R²², R²⁴, R²⁶, R²⁷, R²⁸, R²⁹, and R³⁰ are hydrogen, R²⁴ and R²⁵ are hydroxyl, and the compound is 1,13-dihydroxy-3'-methyl-5'*H*-spiro[dibenzo[c,h]xanthene-7,2'-furan]-5'-one, a salt, a hydrate, a salt hydrate, a stereoisomer, a dehydrate, a tautomer, or a derivative thereof-

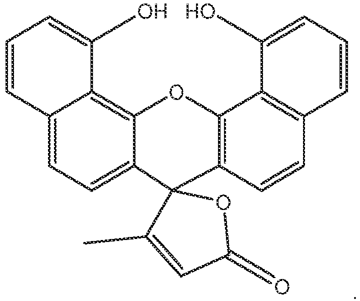

In some embodiments, the compound is an isomer of Formula (IIIA) having a structure according to Formula (IIIii), wherein R¹⁹, R²¹, R²², R²³, R²⁴, R²⁵, R²⁶, R²⁷, R²⁸, and R³⁰ are hydrogen, R²⁰ and R²⁹ are hydroxyl, and the compound is 2,12-dihydroxy-3'-methyl-5'*H-*spiro[dibenzo[a,j]xanthene-14,2'-furan]-5'-one, a salt, a hydrate, a salt hydrate, a stereoisomer, a dehydrate, a tautomer, or a derivative thereof-

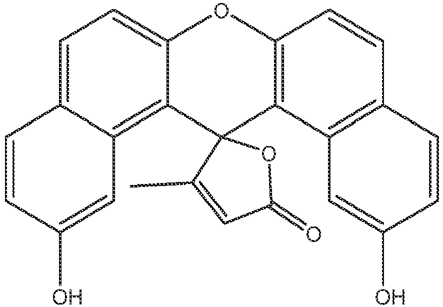

In some embodiments, the compound is a compound or isomer of Formula (IIIA), wherein R¹⁹, R²⁰, R²⁹, and R³⁰ are hydrogen, at least one of R²¹, R²², R²³, and R²⁴ is -N(R')(R"), at least one of R²⁵, R²⁶, R²⁷, and R²⁸ is -N(R')(R"), and any remaining members of R²¹-R²⁸ are hydrogen:

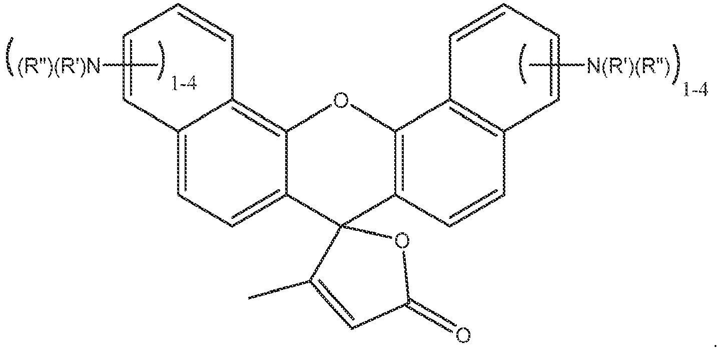

In some embodiments, the compound is a compound or isomer of Formula (III), wherein R³¹ is an unsubstituted C₁ alkenyl, z is a single bond, and R³² is hydrogen. When these substituents are selected for Formula (III), the compound is a compound of Formula (IIIC):

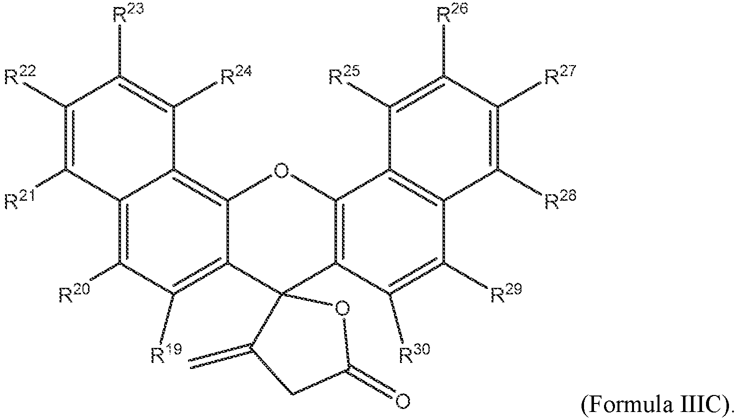

Although Formula (IIIC) is an embodiment of Formula (III), the same substituents may be selected for Formula (IIIi), Formula (IIIii), or Formula (IIIiii), thereby resulting in isomers of Formula (IIIC).

In some embodiments, the compound is a compound or isomer of Formula (III), wherein R³¹ is an unsubstituted C₁ alkyl, z is a double bond, and R³² is an unsubstituted C₁ alkyl. When these substituents are selected for Formula (III), the compound is a compound of Formula (IIID):

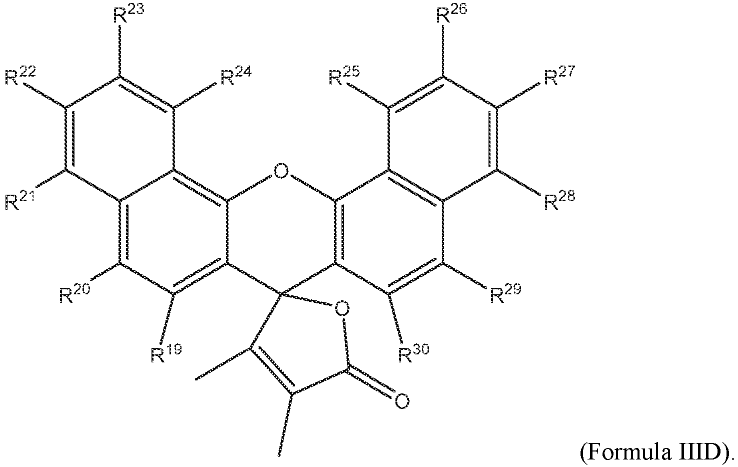

Although Formula (IIID) is an embodiment of Formula (III), the same substituents may be selected for Formula (IIIi), Formula (IIIii), or Formula (IIIiii), thereby resulting in isomers of Formula (IIID).

In some embodiments, the compound is a compound or isomer of Formula (III), wherein R³¹ is hydrogen, z is a double bond, and R³² is hydrogen. When these substituents are selected for Formula (III), the compound is a compound of Formula (IIIE):

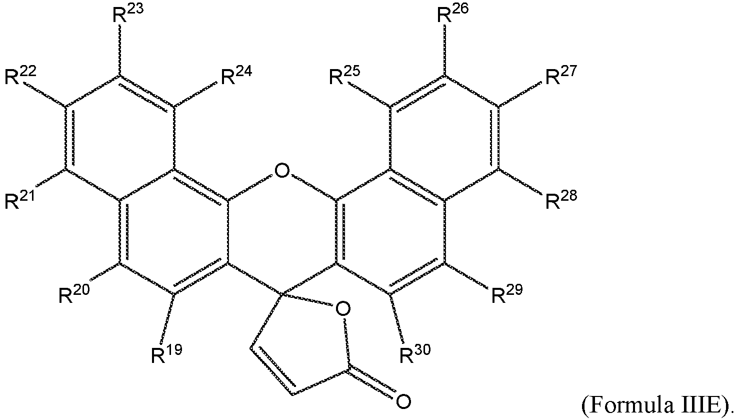

Although Formula (IIIE) is an embodiment of Formula (III), the same substituents may be selected for Formula (IIIi), Formula (IIIii), or Formula (IIIiii), thereby resulting is isomers of Formula (IIIE), such as Formula (IIIiE), Formula (IIIiiE), or Formula (IIIiiiE) below. For example, the compound may be a compound of Formula (IIIi), wherein R³¹ is hydrogen, z is a double bond, and R³² is hydrogen: 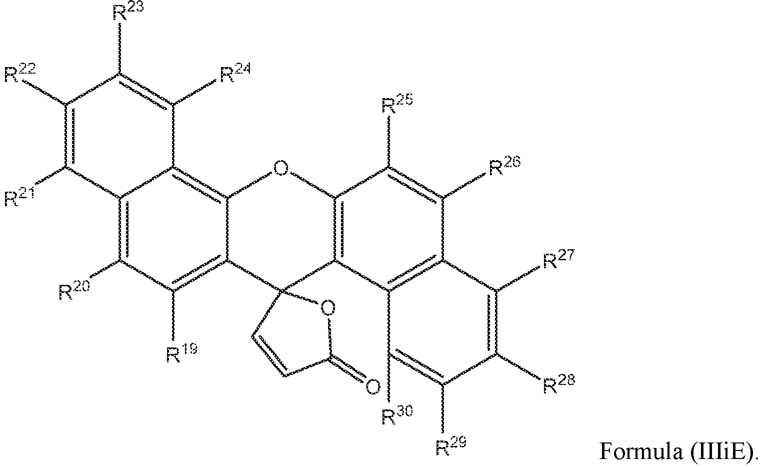 As another example, the compound may be a compound of Formula (IIIii), wherein R³¹ is hydrogen, z is a double bond, and R³² is hydrogen: 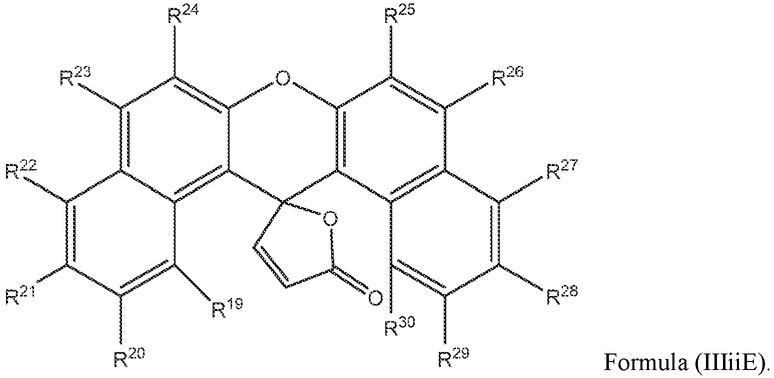 As yet another example, the compound may be a compound of Formula (IIIiii), wherein R³¹ is hydrogen, z is a double bond, and R³² is hydrogen:

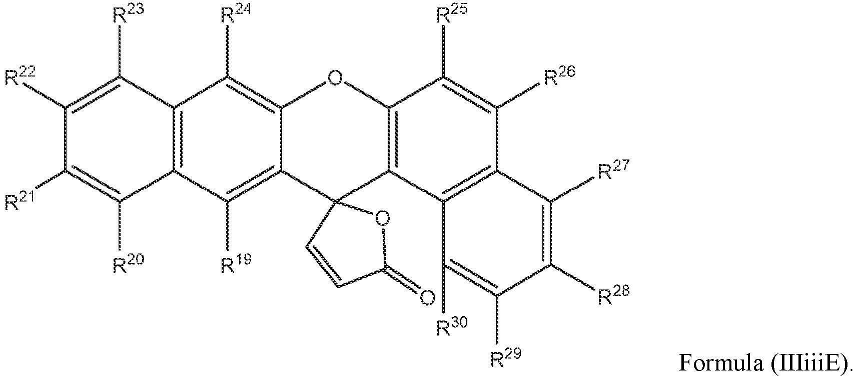

R¹⁹-R³⁰ of the compounds or isomers of Formulas (IIIC), (IIID), and (IIIE) may be selected from those substituents depicted herein for the compounds or isomers of Formula (IIIA). For example, for the compounds or isomers of Formulas (IIIC), (IIID), and (IIIE), R¹⁹, R²⁰, R²¹, R²³, R²⁴, R²⁵, R²⁶, R²⁸, R²⁹, and R³⁰ may be hydrogen, and R²² and R²⁷ may be hydroxyl. When these selections for R¹⁹-R³⁰ are made, for example, for Formula (IIIiE) or Formula (IIIiiE), the resulting compounds, respectively, are [1] (*R*) and/or (*S*) - 4,10-dihydroxy-5'*H-*spiro[dibenzo[a,h]xanthene-14,2'-furan]-5'-one, which has the following structure - 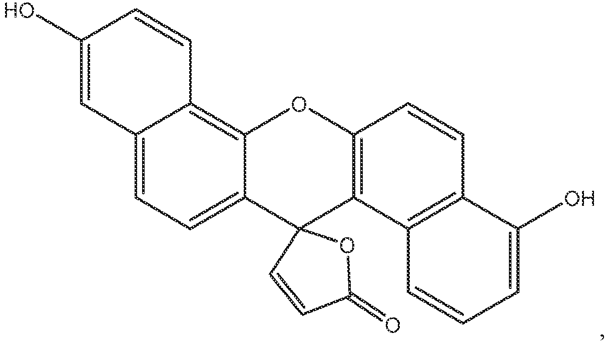 and
[2] 4,10-dihydroxy-5'*H*-spiro[dibenzo[a,j]xanthene-14,2'-furan]-5'-one, which has the following structure -

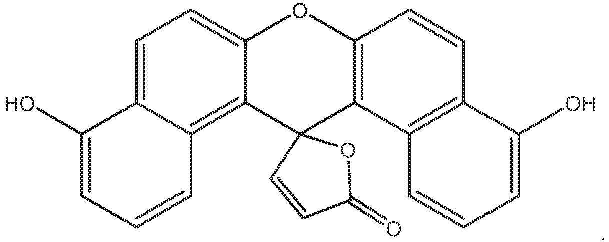

As a further example, for the compounds or isomers of Formulas (IIIC), (IIID), and (IIIE), R¹⁹, R²¹, R²², R²³, R²⁴, R²⁵, R²⁶, R²⁷, R²⁸, and R³⁰ may be hydrogen, and R²⁰ and R²⁹ may be hydroxyl. When these selections for R¹⁹-R³⁰ are made, for example, for Formula (IIIiiE), the resulting compound is 2,12-dihydroxy-5'*H*-spiro[dibenzo[a,j]xanthene-14,2'-furan]-5'-one, which has the following structure -

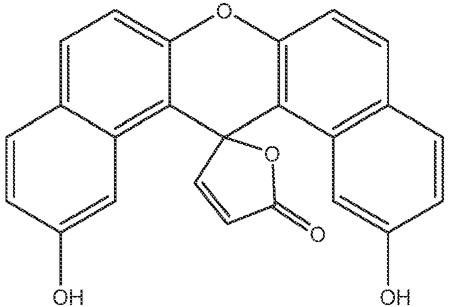

As a still further example, for the compounds or isomers of Formulas (IIIA), (IIIC), (IIID), and (IIIE), R¹⁹, R²⁰, R²¹, R²³, R²⁴, R²⁵, R²⁶, R²⁷, R²⁸, and R³⁰ may be hydrogen, and R²² and R²⁹ may be hydroxyl. When these selections for R¹⁹-R³⁰ are made, for example, for Formula (IIIiiiE), the resulting compound is 2,10-dihydroxy-5'*H*-spiro[dibenzo[a,i]xanthene-14,2'-furan]-5'-one, which has the following structure -

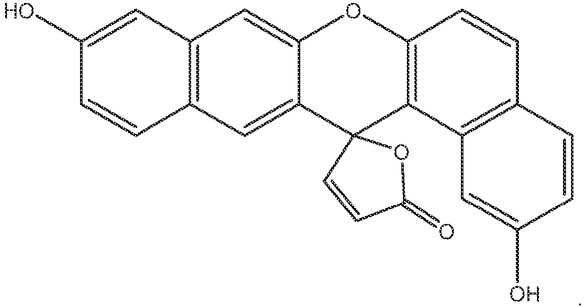

In some embodiments, the compound is a compound or isomer of Formula (IV), wherein R³³, R³⁴, R⁴³, and R⁴⁴ are hydrogen, at least one of R³⁵, R³⁶, R³⁷, and R³⁸ is hydroxyl, and at least one of R³⁹, R⁴⁰, R⁴¹, and R⁴² is hydroxyl:

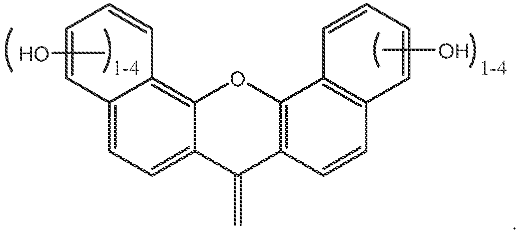

In some embodiments, the compound is a compound or isomer of Formula (IV), wherein R³³, R³⁴, R³⁵, R³⁷, R³⁸, R³⁹, R⁴⁰, R⁴², R⁴³, and R⁴⁴ are hydrogen, R³⁶ and R⁴¹ are hydroxyl, and the compound is 7-methylene-7*H*-dibenzo[c,h]xanthene-3,11-diol, a salt, a hydrate, a salt hydrate, a stereoisomer, a dehydrate, a tautomer, or a derivative thereof - 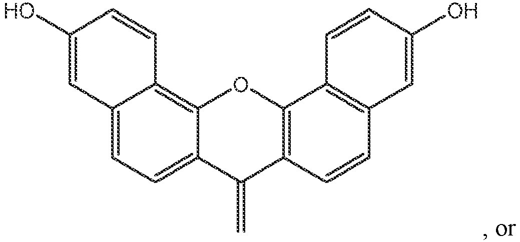 14-methylene-14H-dibenzo[a,h]xanthene-4,10-diol a salt, a hydrate, a salt hydrate, a stereoisomer, a dehydrate, a tautomer, or a derivative thereof -

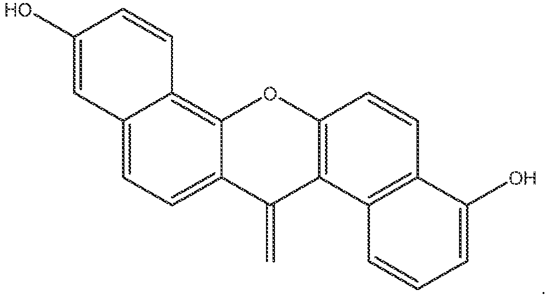

In some embodiments, the compound is a compound or isomer of Formula (IV), wherein R³³, R³⁴, R⁴³, and R⁴⁴ are hydrogen, at least one of R³⁵, R³⁶, R³⁷, and R³⁸ is -N(R')(R"), and at least one of R³⁹, R⁴⁰, R⁴¹, and R⁴² is -N(R')(R"):

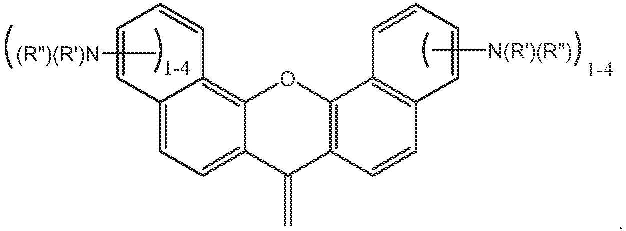

### Polymeric Anti-scale Compositions

Polymer compositions, including anti-scale polymer compositions, are provided herein. The polymer compositions may include a copolymer, which includes a first monomer that is a tagging monomer, and at least one second monomer that is a scale-inhibiting monomer.

In some embodiments, the first monomer is selected from the group consisting of (a) a compound of Formula (I), (b) a compound of Formula (II), (c) a compound or isomer of Formula (III), and (d) a compound or isomer of Formula (IV), which, again, includes salts, hydrates, salt hydrates, stereoisomers, dehydrates, tautomers, or derivatives of the compounds or isomers of Formulas (I), (II), (III), and (IV). In some embodiments, the at least one second monomer includes at least one polymerizable double bond or at least one polymerizable triple bond.

In some embodiments, the copolymers are obtainable by free radical polymerization of two or more types of monomer (including 3, 4, or more different monomers) without restriction on the number of monomer units that are incorporated into the product, provided that at least one of the monomers is a first monomer (i.e., a tagging monomer) and at least one of the monomers is a second monomer (i.e., a scale-inhibiting monomer). In some embodiments, the copolymers include two or more second monomers (i.e., scale-inhibiting units) and one or more first monomers (i.e., tagging units) as described herein.

As used herein, the terms "polymer," "polymers," "polymeric," and the like are used in their ordinary sense as understood by one skilled in the art, and thus may be used herein to refer to or describe a large molecule (or group of such molecules) that contains recurring units (i.e., monomers), including, but not limited to, oligomers, comb polymers, branched polymers, linear polymers, crosslinked polymers, star polymers, etc. Polymers may be formed in various ways, including by polymerizing monomers and/or by chemically modifying one or more recurring units of a precursor polymer. A polymer may be a "copolymer" that includes two or more different recurring units (i.e., monomers) formed by, e.g., copolymerizing two or more different monomers (e.g., 2, 3, 4, 5, 6 or more monomers), and/or by chemically modifying one or more recurring units of a precursor polymer.

The polymers, including copolymers, provided herein are defined in terms of the monomer(s) that form the structures of the polymers. Although, in the interest of clarity, monomers are depicted in isolated, unpolymerized form herein, a person skilled in the art will understand the structural differences between the monomers in unpolymerized and polymerized forms. For example, a person skilled in the art will understand that a polymerized monomer of Formula (II) may have the following structure or a similar structure when polymerized:

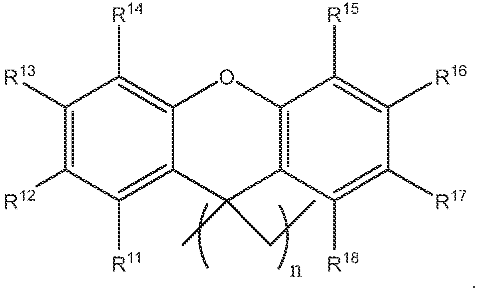

### i. First Monomer

The first monomer of the polymer compositions may include (a) a compound of Formula (I), (b) a compound of Formula (II), (c) a compound or isomer of Formula (III), (d) a compound or isomer of Formula (IV), which, again, may include salts, hydrates, salt hydrates, stereoisomers, dehydrates, tautomers, or derivatives of the compounds or isomers of Formulas (I), (II), (III), and (IV), or (e) a combination thereof. Therefore, the first monomer, for example, may include a salt or salt hydrate of a compound or isomer of Formula (I), (II), (III), or (IV), such as a hydrochloride, dihydrochloride, sulfate, bisulfate, or gluconate salt, or hydrate thereof. As a further example, the first monomer may include a derivative of a compound or isomer of Formula (I), (II), (III), or (IV), such as a derivative formed from the addition of acid and heat to the compound or isomer of Formula (I), (II), (III), or (IV).

### ii. Second Monomer

The at least one second monomer of the polymer compositions provided herein may include any monomer that (i) includes a polymerizable moiety, such as a double bond or a triple bond, and (ii) is a scale inhibitor before and after polymerization, or after polymerization.

In some embodiments, the at least one second monomer is selected from the group consisting of allylsulfonate salts, for example sodium allylsulfonate; acrylic acid; vinyl sulfonic acid; vinyl sulfonate salts; vinyl phosphoric acid; vinyl phosphonate salts; vinylidene diphosphonic acid or salts thereof; methacrylic acid; vinyl acetate; vinyl alcohol; vinyl chloride; unsaturated mono- or di-carboxylic acids or anhydrides, such as maleic anhydride, maleic acid, fumaric acid, itaconic acid, aconitic acid, mesaconic acid, citraconic acid, crotonic acid, isocrontonic acid, angelic acid, and tiglic acid; vinyl chloride; styrene-p-sulfonic acid, or styrene sulfonates salts; acrylamido-2-methylpropanesulfonic acid (AMPS); hydroxyphosphonoacetic acid (HPA); hypophosphorus acids; acrylamides; propargyl alcohol having formula HC≡C-CH₂-OH; butyr-1,4-diol, and mixtures thereof. In some embodiments, two or more types of scale-inhibiting monomer are used as the at least one second monomer; for example, (i) sodium allylsulfonate and maleic acid, (ii) sodium allylsulfonate and maleic anhydride, (iii) sodium allylsulfonate and acrylic acid, or (iv) sodium allylsulfonate, acrylic acid, and at least one of maleic acid or maleic anhydride.

The polymer compositions provided herein generally may include any amount of at least one first monomer and any amount of at least one second monomer. In some embodiments, the first monomer is present in the copolymer at an amount of about 0.01 % to about 10 %, by weight, based on the weight of the copolymer. In some embodiments, the first monomer is present in the copolymer at an amount of about 0.01 % to about 5 %, by weight, based on the weight of the copolymer. In some embodiments, the first monomer is present in the copolymer at an amount of about 0.01 % to about 2 %, by weight, based on the weight of the copolymer. In some embodiments, the first monomer is present in the copolymer at an amount of about 0.01 % to about 1.5 %, by weight, based on the weight of the copolymer. In some embodiments, the first monomer is present in the copolymer at an amount of about 0.01 % to about 1 %, by weight, based on the weight of the copolymer. In some embodiments, the first monomer is present in the copolymer at an amount of about 0.01 % to about 0.75 %, by weight, based on the weight of the copolymer. In some embodiments, the first monomer is present in the copolymer at an amount of about 0.01 % to about 0.5 %, by weight, based on the weight of the copolymer. In some embodiments, the first monomer is present in the copolymer at an amount of about 0.01 % to about 30 %, about 0.01 % to about 20 %, about 0.01 % to about 15 %, about 0.01 % to about 10 %; about 0.01 % to about 8 %; about 0.01 % to about 7 %; about 0.01 % to about 5 %; about 0.01 % to about 3 %, or about 0.01 % to about 2 %, by weight, based on weight of the copolymer.

In some embodiments, the copolymer of a polymer composition has a weight average molecular weight (M_{w}) of about 500 Daltons to about 20,000 Daltons, about 1,200 Daltons to about 15,000 Daltons, about 2,000 Daltons to about 10,000 Daltons, about 2,000 Daltons to about 8,000 Daltons, about 2,000 Daltons to about 6,000 Daltons, about 2,000 Daltons to about 4,000 Daltons, or about 2,000 Daltons to about 3,000 Daltons.

In some embodiments, the polymer compositions may include one or more monomers, groups, or units, as necessary or desired, in addition to the first monomer and at least one second monomer. For example, the polymers may include one or more other groups resulting from a polymerization initiator, end-capping groups, or a combination thereof. In some embodiments, the end capping groups are derived from initiator compounds used in the polymerization of monomers.

The thermal stability of the polymer compositions may be evaluated by heating the polymer in a liquid, for example water or brine, to a temperature, for example, of about 80 °C, about 90 °C, about 100 °C, about 110 °C, about 120 °C, or about 130 °C, and keeping polymer composition in the liquid at that temperature for a period of time, for example, about one week.

In some embodiments, the polymer compositions, including the copolymers provided herein, have a thermal stability such that when a polymer composition is kept at a temperature of about 80 °C in water or brine for about one week, there is less than about a 15 %, about a 10%, about a 5%, about a 4%, or about a 3 % decrease in emission intensity. In some embodiments, the polymer compositions, including the copolymers provided herein, have a thermal stability such that when a polymer composition is kept at a temperature of about 130 °C in water or brine for about one week, there is less than about a 15 %, about a 10%, about a 5%, about a 4%, or about a 3 % decrease in emission intensity. In some embodiments, the water is at a pH of about 7 to about 8. In some embodiments, the brine is natural brine or synthetic brine. In some embodiments, the polymer compositions have a thermal stability such that when a polymer composition is kept at a temperature of about 80 °C in water for about one week, there is less than about a 10%, about a 5%, about a 4%, or about a 3% decrease in emission intensity. In some embodiments, the polymer compositions have a thermal stability such that when a polymer composition is kept at a temperature of about 130 °C in water for about one week, there is less than about a 15%, about a 10% or about a 5% decrease in emission intensity. In some embodiments, the polymer compositions have a thermal stability such that when a polymer composition is kept at a temperature of about 130 °C in water at about pH 8 for about one week, there is less than about a 15%, about a 13%, or about a 10% decrease in emission intensity. In some embodiments, the polymer compositions have a thermal stability such that when a polymer composition is kept at a temperature of about 130 °C in brine for about one week, there is less than about a 20%, about a 15% or about a 10% decrease in emission intensity.

A copolymer, as provided herein, may be present in the polymer compositions at an effective scale-inhibiting amount. As used herein, the phrase "effective scale-inhibiting amount" refers to an amount of a scale-inhibiting copolymer that is effective to provide suitable scale inhibition, removal, reduction, or a combination thereof. In some embodiments, the polymer compositions include an effective scale-inhibiting amount of a copolymer that includes a first monomer and at least one second monomer as described herein. Exemplary scale-inhibiting polymer compositions may, for example, include from about 5 % to about 95 %, by weight, of a scale-inhibiting copolymer that includes a first monomer and at least one second monomer, based on the total weight of the scale-inhibiting polymer composition.

The polymer composition may optionally include one or more additional ingredients, as necessary or desired, such as those described herein, which include water, salts, oils, surfactants, pH adjusting agents (such as acids, bases and buffers), colorants, flow modifiers, other water treatment agents, etc. In some embodiments, the polymer composition consists essentially of a copolymer that includes a first monomer and at least one second monomer, as described herein. When the polymer composition consists essentially of a copolymer that includes a first monomer and at least one second monomer, the polymer composition may include one or more of the foregoing "additional ingredients" and the following "[e]xemplary fluids", because the "additional ingredients" and "[e]xemplary fluids" are non-limiting examples of components that do not materially affect the basic and novel characteristic(s) of the polymer compositions.

In some embodiments, the polymer compositions include (i) a copolymer of a first monomer and at least one second monomer, and (ii) a fluid. Exemplary fluids include those that may be in or intended for industrial water systems or process systems, such as boilers, cooling systems, cooling towers, desalination plants, geothermal power production, irrigation systems, mineral ore extraction systems, paper pulping or manufacturing systems, membrane systems, etc. Other exemplary fluids include fluids for use in the oil industry, such as those for use in the treatment of water injection systems, subsea flow lines, topside production equipment and "down-hole" to control scaling in and around the production well-bore.

In some embodiments, the polymer compositions include an aqueous composition or a water-based fluid, for example a seawater-based fluid. Other fluids, however, are envisioned. In some embodiments, the polymer compositions include a glycol or glycol ether based solvent.

In some embodiments, the polymer compositions include a copolymer of a first monomer and at least one second monomer, as described herein, and, optionally, one or more additional polymers, such as one or more additional scale-inhibiting polymers. The one or more additional polymers may include a tagging agent, and the fluorescence emission of the tagging agent may differ from the fluorescence emission of the first monomer of the copolymer.

In some embodiments, the polymer composition includes one or more copolymers, as described herein, in combination with one or more additional ingredients, such as anionic surfactants (e.g. C₁₀₋₂₀ alkyl benzene sulfonates, C₁₀₋₂₀ olefin sulfonates, C₁₀₋₂₀ alkyl sulfates, C₁₀₋₂₀ alkyl 1 to 25 mole ether sulfates, C₁₀₋₂₀ paraffin sulfonates, C₁₀₋₂₀ soaps, C₁₀₋₂₀ alkyl phenol sulfates, sulfosuccinates, sulfosuccinamates, lignin sulfonates, fatty ester sulfonates, C₁₀₋₂₀ alkyl phenyl ether sulfates, C₁₀₋₂₀ alkyl ethanolamide sulfates, C₁₀₋₂₀ alpha sulfo fatty acid salts, C₁₀₋₂₀ acyl sarcosinates, isethionates, C₁₀₋₂₀ acyl taurides, C₁₀₋₂₀ alkyl hydrogen phosphates), non-ionic surfactants (e.g. ethoxylated and/or propoxylated C₁₀₋₂₀ alcohols, ethoxylated and/or propoxylated C₁₀₋₂₀ carboxylic acids, alkanolamides, amine oxides, and/or C₁₀₋₂₀ acyl sorbitan and/or glyceryl ethoxylates), amphoteric surfactants (e.g. betaines, sulfobetaines, and/or quaterised imidazolines), and/or cationic surfactants (e.g. benzalkonium salts, C₁₀₋₂₀ alkyl trimethyl ammonium salts, and/or C₁₀₋₂₀ alkyl trimethyl); sequestrants; chelating agents; corrosion inhibitors (e.g., imidazoline and quaterantry ammonium salts); and/or other threshold agents (e.g. polymers such as aminometholine phosphonate polymers, polyacrylic acid, or non polymeric agents such as sodium tripolyphosphate, sodium ethylenediamine tetracetate, sodium nitrilo triacetate, tetra potassium pyrophosphate, acetodiphosphonic acid and its salts, ammonium trismethylene phosphonic acid and its salts, ethylenediamine tetrakis (methylene phosphonic) acid and its salts, diethylenetriamine pentakis (methylene phosphonic) acid and its salts); tolyltriazole and mixtures of nitrate, benzqate, HHP and/or PTCB); hydrate inhibitors (e.g., methanol); cinetic inhibitors such as anti-agglomeration agents; biocides (e.g. tetrakis (hydroxymethyl) phosphonium salts, formaldehyde, glutaraldehyde, DENPA, bromopol isothiazoronal); oxidising biocides and/or bleaches (e.g. chlorine, chlorine dioxide, hydrogen peroxide, sodium perborate); foam controlling agents, such as silicone antifoams; oxygen scavengers such as hydrazines and/or hydroxylamines; pH controlling and/or buffering agents, such as amines, borates, citrates and/or acetates; chromium salts; zinc salts; asphaltene inhibitors; wax inhibitors; demulsifiers; other scale inhibitors; and/or other water treatment agents such as polymeric dispersants and coagulants including polymaleic, polyacrylic and polyvinylsulfonic acids and their salts, starches and/or carboxy methyl cellulose, and/or molybdates.

In some embodiments, the polymer composition includes two or more copolymers. When two or more copolymers are present, each copolymer may include a different first monomer, and each of the different first monomers may exhibit a different fluorescence emission. For example, a polymer composition may include (i) a first copolymer including a first monomer, such as resorcinmalein, which has a fluorescence emission maximum of about 500 to about 520 nm, (ii) a second copolymer including a first monomer, such as one based on diethylaminophenol, which has a fluorescence emission maximum of about 550 nm to about 590 nm, (iii) a third copolymer including a first monomer, such as naphthomalein, which has a fluorescence emission maximum of about 640 nm to about 680 nm, or (iv) a combination thereof. Such a polymer composition also may include an additional copolymer including a first monomer, such as hydroxyjulolidine, which has a fluorescence emission maximum of about 570 nm to about 600 nm. The differences in fluorescence emission maximum may permit the methods described herein to be used to determine an amount of each copolymer present in a fluid or system, the differences between the amounts of each copolymer in a fluid or system, or a combination thereof.

In some embodiments, the polymer compositions include about 5 % to about 95 %, by weight, of a copolymer of a first monomer and at least one second monomer, as described herein, and about 5 % to about 90 %, by weight, of one or more of any of the additional ingredients described herein, based on the total weight of a polymer composition.

A copolymer of at least one first monomer and at least one second monomer may be combined with water using any suitable method. For example, a copolymer may be dissolved, suspended, dispersed, or emulsified in water. The amount of water in an aqueous polymer composition may vary, as necessary or desired. For example, an aqueous polymer composition may include about 20 % to about 80 %, by weight, of a copolymer of a first monomer and a second monomer, as described herein, based on the total weight of the aqueous polymer composition.

In some embodiments, the pH of a polymer composition may be such that the acidic functionalities of a copolymer, as described herein, are neutralized. For example, the composition may be neutralized by adjusting the pH of the composition to a pH in a range of about 2 to about 13.

### Methods of Synthesis

The compounds or isomers of Formula (I), (II), (III), and (IV) may be synthesized with any technique, including those provided herein.

In some embodiments, the compounds or isomers of Formula (I), (II), (III), and (IV) are formed via a condensation reaction. The condensation reaction may include contacting an aryl alcohol, a condensation catalyst, and a compound according to formula (A) to form the condensation product. The condensation product may include a compound or isomer of Formula (I), (II), (III), or (IV), which, as explained herein, includes the salts, hydrates, salt hydrates, stereoisomers, dehydrates, tautomers, and derivatives of the compounds or isomers of Formula (I), Formula (II), Formula (III), and Formula (IV).

The compound according to Formula (A) has the following structure: 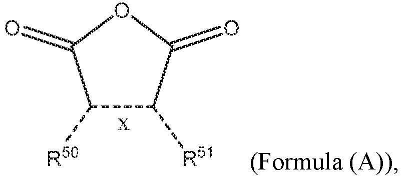 wherein R⁵⁰ and R⁵¹ are independently selected from the group consisting of hydrogen, hydroxyl, C₁-C₆ alkyl, and C₁-C₆ alkenyl, and x is a single bond or double bond.

In some embodiments, R⁵⁰ is an unsubstituted C₁ alkyl, x is a double bond, R⁵¹ is hydrogen, and the compound of Formula (A) is 3-methylfuran-2,5-dione -

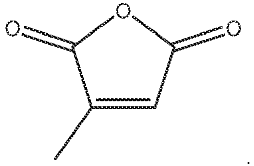

In some embodiments, R⁵⁰ is hydrogen, x is a single bond, R⁵¹ is an unsubstituted C₃-alk-1-enyl, and the compound of Formula (A) is 3-allyldihydrofuran-2,5-dione -

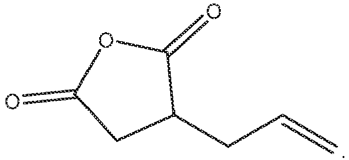

In some embodiments, R⁵⁰ and R⁵¹ are hydrogen, x is a double bond, and the compound of Formula (A) is furan-2,5-dione -

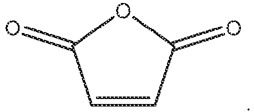

In some embodiments, R⁵⁰ is hydrogen, x is a single bond, R⁵¹ is an unsubstituted C₁-alkenyl, and the compound of Formula (A) is 3-methylenedihydrofuran-2,5-dione -

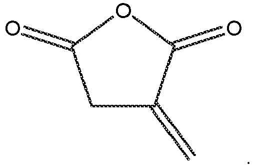

In some embodiments, R⁵⁰ and R⁵¹ are an unsubstituted C₁ alkyl, x is a double bond, and the compound of Formula (A) is 3,4-dimethylfuran-2,5-dione -

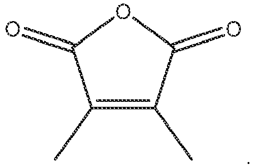

As used herein, the phrase "aryl alcohol" generally refers to a compound that includes (i) an aryl moiety, and (ii) at least one hydroxyl moiety. In some embodiments, the aryl alcohol includes (i) an aryl moiety, and (ii) two hydroxyl moieties. In some embodiments, the aryl alcohol is resorcinol. In some embodiments, the aryl alcohol is 1,6-dihydroxynaphthalene. The aryl alcohol, however, may include any compound that is capable of forming a compound or isomer of Formula (I), (II), (III), or (IV). For example, when the methods provided herein are used to produce a compound or isomer of Formula (I), the aryl alcohol may be of the following formula: 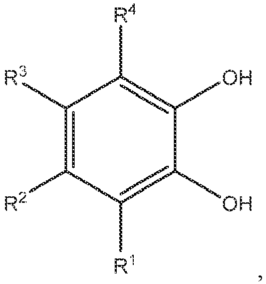 wherein R¹, R², R³, and R⁴ are as defined herein.

The contacting of the aryl alcohol, the condensation catalyst, and the compound of Formula (A) may occur at any temperature and/or pressure that is effective to form the condensation product. In some embodiments, the contacting of the aryl alcohol, the condensation catalyst, and the compound of Formula (A) occurs at a temperature of about 50 °C to about 150°C, about 75 °C to about 150°C, about 100 °C to about 150°C, or about 100 °C to about 125 °C. In some embodiments, the contacting of the aryl alcohol, the condensation catalyst, and the compound of Formula (A) occurs at ambient pressure, and a temperature of about 50 °C to about 150°C, about 75 °C to about 150 °C, about 100 °C to about 150°C, or about 100 °C to about 125 °C.

The condensation catalyst may include any catalyst capable of effecting the condensation of the aryl alcohol and the compound of Formula (A). In some embodiments, the condensation catalyst is a Lewis acid. Non-limiting examples of Lewis acids include ZnCl₂, FeCl₃, AlCl₃, and BCl₃. In some embodiments, the condensation catalyst is a sulfonic acid. The sulfonic acid may include an C₁-C₆ alkyl sulfonic acid, a C₅-C₁₄ aryl sulfonic acid, or a combination thereof. In some embodiments, the C₁-C₆ alkyl sulfonic acid is methanesulfonic acid (MeSO₃H). In some embodiments, the C₅-C₁₄ aryl sulfonic acid is p-toluenesulfonic acid.

Also provided herein are methods of polymerizing a compound or isomer of Formula (I), (II), (III), or (IV). In some embodiments, the methods include contacting a compound or isomer of Formula (I), (II), (III), or (IV) (e.g., a condensation product of the foregoing methods) with at least one second monomer to form a copolymer, wherein the at least one second monomer includes a polymerizable double bond or triple bond. The at least one second monomer may be contacted with an amount of the condensation product effective to produce a copolymer that includes a desirable amount of the condensation product as described herein, for example, such as about 0.01 % to about 5 %, or about 0.01 % to about 2 %, by weight, based on the weight of the copolymer.

The at least one second monomer generally may include any monomer that is polymerizable due to the presence of a polymerizable double bond or triple bond. The phrases "polymerizable double bond", "polymerizable triple bond", and the like refer to bonds that may react with a functional group of at least one other monomer (e.g., under conditions described herein) to form a polymer. The at least one second monomer may be a scale-inhibitor alone and/or when polymerized. In some embodiments, the at least one second monomer includes sodium allyl sulfonate and at least one of maleic acid, maleic anhydride, or acrylic acid. In some embodiments, the at least one second monomer includes a compound of Formula (V) - 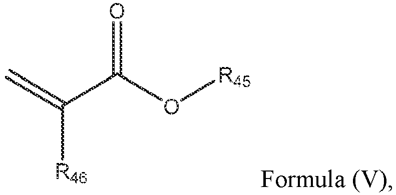 wherein R⁴⁵ and R⁴⁶ are independently selected from the group consisting of hydrogen and C₁-C₆ alkyl. In some embodiments, R⁴⁶ and R⁴⁵ are hydrogen. In some embodiments, R⁴⁶ is an unsubstituted C₁ alkyl, and R⁴⁵ is hydrogen.

The polymer compositions provided herein generally may be prepared by any polymerization method. For example, a free-radical polymerization method may be employed. Other exemplary methods include aqueous bulk/dispersion polymerization, solution polymerization, or emulsion polymerization. In some embodiments, the polymerization process is a solution polymerization, wherein water is charged to a reaction vessel fitted with a mechanical stirrer and water condenser, and heated to a temperature within a range of about 45 °C to about 150° C, or about 45 °C to about 110 °C. One or more polymerization initiators may be added to the reactor. The choice of initiator may inform the temperature at which the reaction is performed. A first monomer may be added to the reactor, added to a monomer feed or fed separately. A monomer feed(s), soluble initiator feed, and optionally a chain transfer reagent feed may be added to a vessel at a predetermined time or over time.

In some embodiments, the polymerization of monomers, including at least one first monomer and at least one second monomer, is achieved in the presence of one or more polymerization initiators including, but not limited to, inorganic peroxides, for example ammonium persulfate (APS), hydroxymethanesulfinic acid monosodium salt dehydrate, potassium persulfate, and sodium persulfate; organic peroxides, for example tert-butyl hydroperoxide (TBHP), tert-butyl peracetate, cumene hydroperoxide, 2,5-Di(tert-butylperoxy)-2,5-dimethyl-3-hexyne, dicumyl peroxide, 2,5-bis(tert-butylperoxy)-2,5-dimethylhexane, 2,4-pentanedione peroxide, 1,1-bis(tert-butylperoxy)-3,3,5-trimethylcyclohexane, 1,1-bis(tert-butylperoxy)cyclohexane, 1,1-bis(tert-amylperoxy)cyclohexane, benzoyl peroxide, 2-butanone peroxide, tert-butyl peroxide, lauroyl peroxide, tert-butyl peroxybenzoate, and tert-butylperoxy 2-ethylhexyl carbonate; azo compounds, for example azobisisobutyronitrile (AIBN), 4,4'-azobis(4-cyanovaleric acid), 1,1'-azobis(cyclohexanecarbonitrile), 2,2'-azobis(2-methylpropionamidine) dihydrochloride, and 2,2'-azobis(2-methylpropionitrile); tetrakis(hydroxymethyl)phosphonium sulfate (THPS); cerium ammonium nitrate; perchlorates; triphenylphosphine; and the like, and compositions or mixtures including one or more of these initiators. In some embodiments, the initiator is selected from the group consisting of ammonium persulfate, tert-butyl hydroperoxide, and 4,4'-azobis(4-cyanovaleric acid).
Polymerization initiators generally may be used at an amount of about 0.01 % to about 10 %, by weight, based on the total weight of the monomers. Polymerization initiators may be used in conjunction with heat to initiate polymerization of monomers. In some embodiments, two or more initiators are used; for example, an inorganic peroxide and an organic peroxide. In some embodiments, ammonium persulfate (APS) and an organic peroxide are used to initiate polymerization. The initiator or initiators used to achieve polymerization may affect the physical properties of the resulting polymer. The initiator or initiators may be added to a polymerization reaction mixture, for example, at the start of the reaction, at various times during the polymerization, and/or gradually over time, e.g., over several minutes or hours. If two or more initiators are used, then the initiators may be dosed simultaneously or sequentially during polymerization. In some embodiments, one initiator is dosed at the start of polymerization, at various times during polymerization, and/or gradually over time, and a different initiator is used at later stages the polymerization.

### Fluorescent Tracers

As used herein, the phrase "fluorescent tracer" refers to a fluorescent compound, which, upon or after excitation at a wavelength, re-emits energy at a wavelength that is different than the excitation wavelength. In some embodiments, the fluorescent tracer has an emission lifetime of about 2 nanoseconds to about 1000 microseconds. In some embodiments, one type of fluorescent tracer is used in the systems and methods provided herein. In some embodiments, two or more types of fluorescent tracer are used in the systems and methods provided herein, wherein at least two of the fluorescent tracers are different.

A fluorescent tracer may be combined with one or more other components of an anti-scalant composition in any manner. For example, a fluorescent tracer may be dispersed in one or more other components of an anti-scalant composition, chemically bonded (e.g., covalently, ionically, etc.) to one or more other components of an anti-scalant composition, or a combination thereof. For instance, a fluorescent tracer may be covalently bonded to a monomer of a polymer, thereby constituting at least part of a pendant group of the polymer. When a fluorescent tracer is polymerized alone, or with one or more other monomers, the fluorescent tracer is a monomer tag.

In some embodiments, the fluorescent tracer includes a xanthene fluorophore. As used herein the phrase "xanthene fluorophore" refers to xanthene or a derivative of xanthene, such as a substituted derivative.

Non-limiting examples of fluorescent tracers that may be used in the systems and methods herein include 3-*N*,3-*N*,6-*N*,6-*N*-tetramethylacridine-3,6-diamine (acridine orange)(CAS Registry No. 65-61-2); 2-anthracenesulfonic acid sodium salt (CAS Registry No. 16106-40-4); 1,5-anthracenedisulfonic acid (CAS Registry No. 61736-91-2) and salts thereof; 2,6-anthracenedisulfonic acid (CAS Registry No. 61736-95-6) and salts thereof; 1,8-anthracenedisulfonic acid (CAS Registry No. 61736-92-3) and salts thereof; anthra[9,1,2-cde]benzo[rst]pentaphene-5,10-diol, 16,17-dimethoxy-, bis(hydrogen sulfate), disodium salt, also known as Anthrasol Green IBA (CAS Registry No. 2538-84-3); bathophenanthrolinedisulfonic acid disodium salt (CAS Registry No. 52746-49-3); amino 2,5-benzene disulfonic acid (CAS Registry No. 41184-20-7); 2-(4-aminophenyl)-6-methylbenzothiazole (CAS Registry No. 92-36-4); 1H-benz[de]isoquinoline-5-sulfonic acid, 6-amino-2,3-dihydro-2-(4-methylphenyl)-1,3-dioxo-, monosodium salt, i.e., Brilliant Acid Yellow 8G (CAS Registry No. 2391-30-2, i.e., Lissamine Yellow FF, Acid Yellow 7); phenoxazin-5-ium, 1-(aminocarbonyl)-7-(diethylamino)-3,4-dihydroxy-, chloride, also known as Celestine Blue (CAS Registry No. 1562-90-9); 9-amino-5-imino-5H-benzo[a]phenoxazine acetate salt, also known as cresyl violet acetate (CAS Registry No. 10510-54-0); 4-dibenzofuransulfonic acid (CAS Registry No. 42137-76-8); 3-dibenzofuransulfonic acid (CAS Registry No. 215189-98-3); 1-ethylquinaldinium iodide (CAS Registry No. 606-53-3); fluorescein (CAS Registry No. 2321-07-5); fluorescein, sodium salt (CAS Registry No. 518-47-8, i.e.,Acid Yellow 73, Uranine); Keyfluor White ST (CAS Registry No. 144470-48-4, aka Flu. Bright 28); benzenesulfonic acid, 2,2'-(1,2-ethenediyl)bis[5-[[4-[bis(2-hydroxyethyl)amino]-6-[(4-sulfophenyl)amino]-1,3,5-triazin-2-yl]amino]-, tetrasodium salt, i.e., Keyfluor White CN (CAS Registry No. 16470-24-9); C.I. Fluorescent Brightener 230, i.e., Leucophor BSB (CAS Registry No. 68444-86-0); benzenesulfonic acid, 2,2'-(1,2-ethenediyl)bis[5-[[4-[bis(2-hydroxyethyl)amino]-6-[(4-sulfophenyl) amino]-1,3,5-triazin-2-yl]amino]-, tetrasodium salt, i.e., Leucophor BMB (CAS Registry No. 16470-24-9, i.e., Leucophor U, Flu. Bright. 290); 9,9'-biacridinium, 10,10'-dimethyl-, dinitrate, i.e., Lucigenin (CAS Registry No. 2315-97-1, i.e., bis-N-methylacridinium nitrate); 1-deoxy-1-(3,4-dihydro-7,8-dimethyl-2,4-dioxobenzo[g]pteridin-10(2H)-yl)-D-ribitol, i.e., Riboflavin or Vitamin B2 (CAS Registry No. 83-88-5); mono-, di-, or tri-sulfonated napthalenes, including but not limited to 1,5-naphthalenedisulfonic acid, disodium salt (hydrate) (CAS Registry No. 1655-29-4, i.e., 1,5-NDSA hydrate), 2-amino-1-naphthalenesulfonic acid (CAS Registry No. 81-16-3), 5-amino-2-naphthalenesulfonic acid (CAS Registry No. 119-79-9), 4-amino-3-hydroxy-1-naphthalenesulfonic acid (CAS Registry No. 90-51-7), 6-amino-4-hydroxy-2-naphthalenesulfonic acid (CAS Registry No. 116-63-2), 7-amino-1,3-naphthalenesulfonic acid, potassium salt (CAS Registry No. 79873-35-1), 4-amino-5-hydroxy-2,7-naphthalenedisulfonic acid (CAS Registry No. 90-20-0), 5-dimethylamino-1-naphthalenesulfonic acid (CAS Registry No. 4272-77-9), 1-amino-4-naphthalene sulfonic acid (CAS Registry No. 84-86-6), 1-amino-7-naphthalene sulfonic acid (CAS Registry No. 119-28-8), and 2,6-naphthalenedicarboxylic acid, dipotassium salt (CAS Registry No. 2666-06-0); 3,4,9,10-perylenetetracarboxylic acid (CAS Registry No. 81-32-3); C.I. Fluorescent Brightener 191, i.e., Phorwite CL (CAS Registry No. 12270-53-0); C.I. Fluorescent Brightener 200, i.e., Phorwite BKL (CAS Registry No. 61968-2-7); benzenesulfonic acid, 2,2'-(1,2-ethenediyl)bis[5-(4-phenyl-2H-1,2,3-triazol-2-yl)-, dipotassium salt, i.e., Phorwite BHC 766 (CAS Registry No. 52237-03-3); benzenesulfonic acid, 5-(2H-naphtho[1,2-d]triazol-2-yl)-2-(2-phenylethenyl)-, sodium salt, i.e., Pylaklor White S-15A (CAS Registry No. 6416-68-8); 1,3,6,8-pyrenetetrasulfonic acid, tetrasodium salt (CAS Registry No. 59572-10-0); pyranine, (CAS Registry No. 6358-69-6, i.e., 8-hydroxy-1,3,6-pyrenetrisulfonic acid, trisodium salt); quinoline (CAS Registry No. 91-22-5); 3H-phenoxazin-3-one, 7-hydroxy-, 10-oxide, i.e., Rhodalux (CAS Registry No. 550-82-3); xanthylium, 9-(2,4-dicarboxyphenyl)-3,6-bis(diethylamino)-, chloride, disodium salt, i.e., Rhodamine WT (CAS Registry No. 37299-86-8); phenazinium, 3,7-diamino-2,8-dimethyl-5-phenyl-, chloride, i.e., Safranine O (CAS Registry No. 477-73-6); C.I. Fluorescent Brightener 235, i.e., Sandoz CW (CAS Registry No. 56509-06-9); benzenesulfonic acid, 2,2'-(1,2-ethenediyl)bis[5-[[4-[bis(2-hydroxyethyl)amino]-6-[(4-sulfophenyl)amino]-1,3,5-triazin-2-yl] amino]-, tetrasodium salt, i.e., Sandoz CD (CAS Registry No. 16470-24-9, i.e., Flu. Bright. 220); benzenesulfonic acid, 2,2'-(1,2-ethenediyl)bis[5-[[4-[(2-hydroxypropyl)amino]-6-(phenylamino)-1,3,5-triazin-2-yl]amino]-, disodium salt, i.e., Sandoz TH-40 (CAS Registry No. 32694-95-4); xanthylium, 3,6-bis(diethylamino)-9-(2,4-disulfophenyl)-, inner salt, sodium salt, i.e., Sulforhodamine B (CAS Registry No. 3520-42-1, i.e., Acid Red 52); benzenesulfonic acid, 2,2'-(1,2-ethenediyl)bis[5-[[4-[(aminomethyl)(2-hydroxyethyl)amino]-6-(phenylamino)-1,3,5-triazin-2-yl] amino]-, disodium salt, i.e., Tinopal 5BM-GX (CAS Registry No. 169762-28-1); Tinopol DCS (CAS Registry No. 205265-33-4); benzenesulfonic acid, 2,2'-([1,1'-biphenyl]-4,4'-diyldi-2,1-ethenediyl)bis-, disodium salt, i.e., Tinopal CBS-X (CAS Registry No. 27344-41-8); benzenesulfonic acid, 5-(2H-naphtho[1,2-d]triazol-2-yl)-2-(2-phenylethenyl)-, sodium salt, i.e., Tinopal RBS 200, (CAS Registry No. 6416-68-8); 7-benzothiazolesulfonic acid, 2,2'-(1-triazene-1,3-diyldi-4,1-phenylene)bis[6-methyl-, disodium salt, i.e., Titan Yellow (CAS Registry No. 1829-00-1, i.e., Thiazole Yellow G); an ammonium salt, a potassium salt, a sodium salt, or a combination thereof.

When a fluorescent tracer is a salt, the fluorescent tracer may include one or more types of counterions. For example, if a fluorescent tracer includes Na⁺ as a counterion, then the fluorescent tracer may include, additionally or alternatively, one or more counterions other than Na⁺, such as K⁺, Li⁺, NH₄ ⁺, Ca²⁺, Mg²⁺, etc. As a further example, if a fluorescent tracer includes Cl⁻ as a counterion, then the fluorescent tracer may include, additionally or alternatively, one or more counterions other than Cl-, such as SO₄ ²⁻, PO₄ ³⁻, HPO₄ ²⁻, H₂PO₄ ⁻, CO₃ ²⁻, HCO₃ ⁻, etc.

A fluorescent tracer may be modified in any manner to obtain one or more desired characteristics. For example, a desired molecular weight and/or physical size of a fluorescent tracer may be achieved by bonding a fluorescent tracer to a polymeric molecule, incorporating a fluorescent tracer into a microsphere, adding one or more chemical moieties to a fluorescent tracer, or a combination thereof. Other modifications are envisioned.

### Fluid Treatment Systems

The methods and systems provided herein may include or be used to monitor a variety of fluid treatment systems, including aqueous systems. Non-limiting examples of such systems include boiler water, cooling water, seawater (e.g., in oil platform applications), membrane and thermal desalination, brackish water, oilfield water (e.g., topside and/or downhole), biogas, municipal treatment plant water, and industrial treatment plant water systems.

For example, the methods and systems herein may include or be used to monitor oilfield injection and production waters. In some embodiments, the systems and methods herein are utilized in a squeeze application.

In some embodiments, the methods and systems herein include or are used to monitor a boiler water system. In some embodiments, the methods and systems herein include or are used to monitor a cooling water system.

In some embodiments, the methods and systems herein include or are used to monitor a brackish water, reuse water, or seawater system.

In some embodiments, the methods and systems herein include or are used to monitor an oilfield water system. The oilfield water may be downhole water that is pumped underground (e.g., for enhanced oil recovery) and/or may be used to treat topside oilfield water.

In some embodiments, the methods and systems herein include or are used to monitor a municipal water treatment system.

Generally, the methods and systems herein include or are used to monitor a fluid in oil or gas applications, for example water injection, production zones, top-side operations, pipelines, and tankage; in pulp or paper applications, for example digestors, headbox, showers and bleach plants; in municipal or industrial applications, for example desalination, cooling towers, sugar refining, and waste treatment; and in metals or mining applications, for example heap leaching, carbon circuits, slurry transport, and digestors.

In some embodiments, the methods and systems herein include or are used to monitor a reverse osmosis system.

## Claims

1. A system for monitoring and/or controlling dosing of an anti-scalant, the system comprising:
an analyzer comprising a fluorometer;
a controller comprising a processor and a memory unit;
a dosing algorithm stored by the memory unit of the controller;
a pump configured to add an amount of an anti-scalant composition into a fluid stream of a fluid treatment system; and
a reservoir in fluid communication with the pump, wherein the anti-scalant composition is disposed in the reservoir;
wherein the processor is configured to (i) receive from the analyzer a first signal comprising fluorometry data collected by the fluorometer from (a) an inlet stream, (b) an outlet stream, or (c) a combination thereof of the fluid treatment system, (ii) determine a dosing instruction by applying the fluorometry data to the dosing algorithm, and (iii) provide a second signal comprising the dosing instruction to the pump,
wherein the pump is configured to modify, based on the dosing instruction, the amount of the anti-scalant composition that is added to the fluid stream of the fluid treatment system, and
wherein the anti-scalant composition is a polymer composition comprising the tagging monomer, wherein the polymer composition comprises-
a copolymer comprising -
(i) a first monomer selected from the group consisting of (a) a compound of Formula (I), (b) a compound of Formula (II), (c) a compound or isomer of Formula (III), and (d) a compound or isomer of Formula (IV), wherein the first monomer is the tagging monomer, and
(ii) at least one second monomer comprising at least one polymerizable double bond or at least one polymerizable triple bond, wherein the at least one second monomer is a scale-inhibiting monomer;
wherein the first monomer is present in the copolymer at an amount of about 0.01 % to about 10 %, by weight, based on the weight of the copolymer; 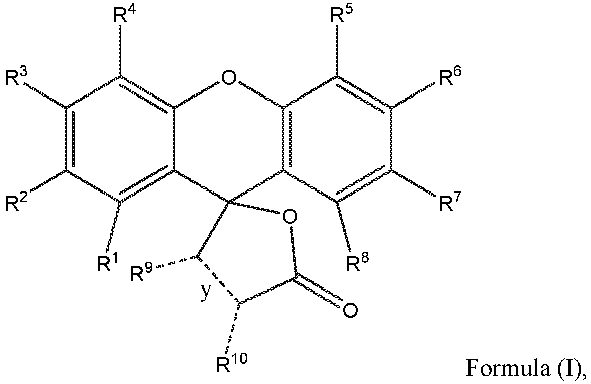 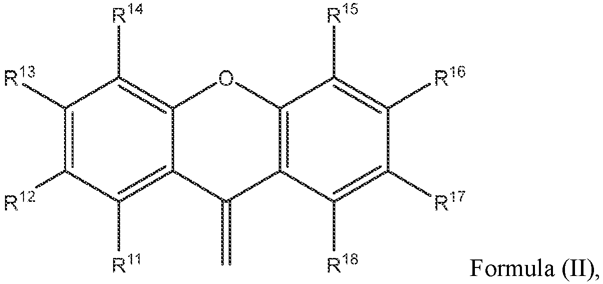 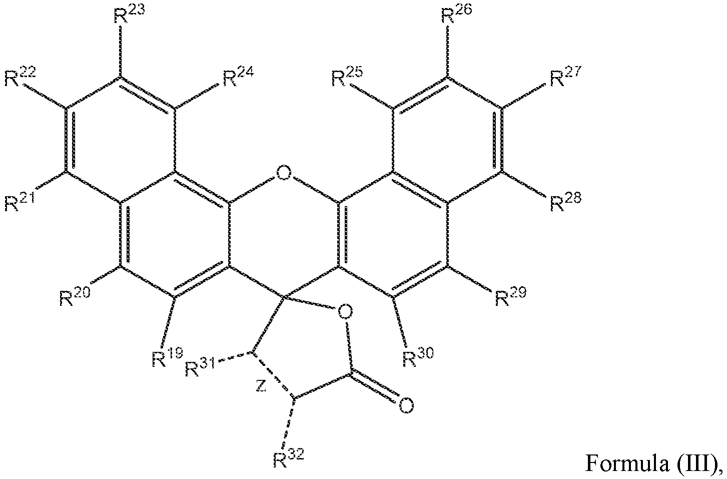 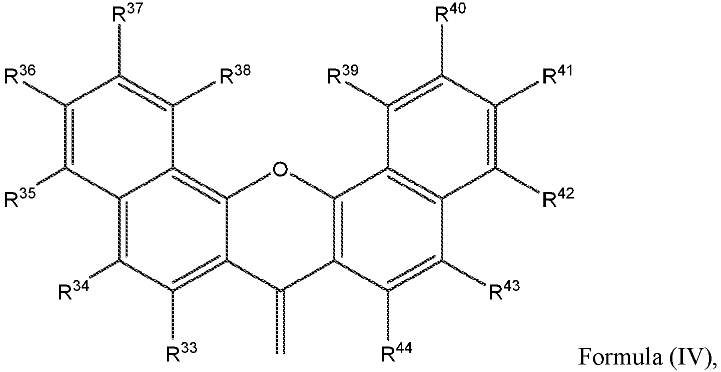
wherein R¹, R², R³, R⁴, R⁵, R⁶, R⁷, R⁸, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁰, R²⁷, R²⁸, R²⁹, R³⁰, R³³, R³⁴, R³⁵, R³⁶, R³⁷, R³⁸, R³⁹, R⁴⁰, R⁴¹, R⁴², R⁴³, and R⁴⁴ are independently selected from the group consisting of hydrogen, hydroxyl, C₁-C₆ alkoxy, C₂-C₆ alkenoxy, C₂-C₆ alkynoxy, -N(R')(R"), C₁-C₆ alkyl, C₂-C₆ alkenyl, C₂-C₆ alkynyl, and C₄-C₁₄ aryl,
wherein R' and R" are independently selected from the group consisting of hydrogen and C₁-C₆ alkyl,
wherein R⁹, R¹⁰, R³¹, and R³² are independently selected from the group consisting of hydrogen, hydroxyl, C₁-C₆ alkyl, and C₁-C₆ alkenyl,
wherein y is a single bond or a double bond,
wherein (i) y is a double bond or (ii) R⁹ is a C₁ alkenyl,
wherein z is a single bond or a double bond, and
wherein (i) z is a double bond or (ii) R³¹ is a C₁ alkenyl;
wherein the isomers of Formula (III) comprise a compound of Formula (IIIi), a compound of Formula (IIIii), a compound of Formula (IIIiii), or a combination thereof - 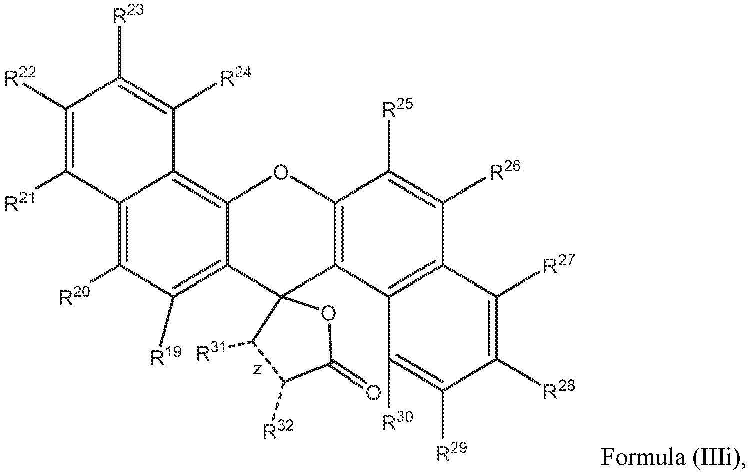 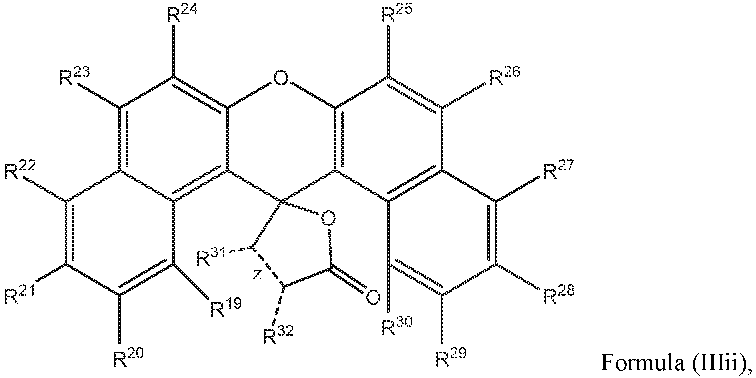 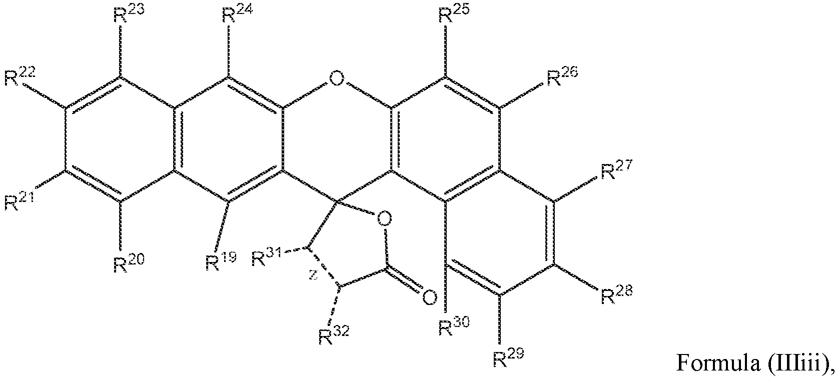 and
wherein the isomers of Formula (IV) comprise a compound of Formula (IVi), a compound of Formula (IVii), a compound of Formula (IViii), or a combination thereof -

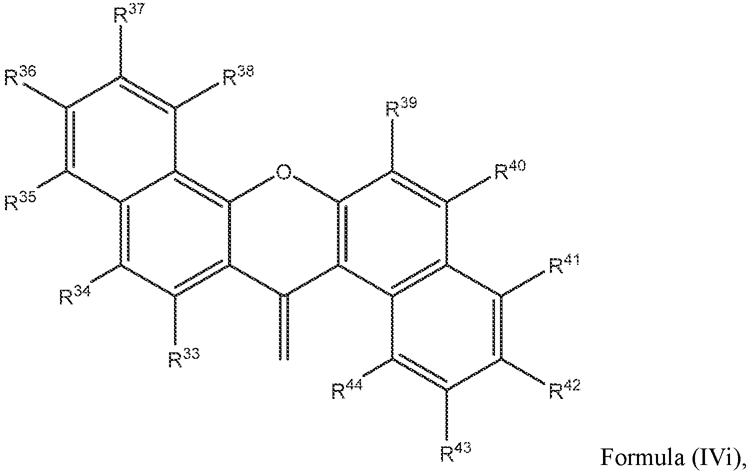

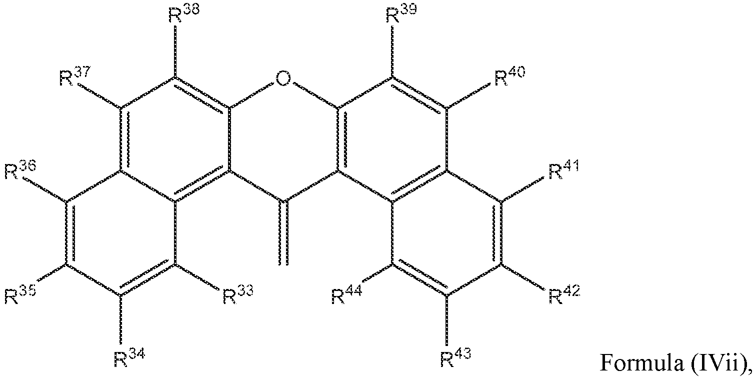

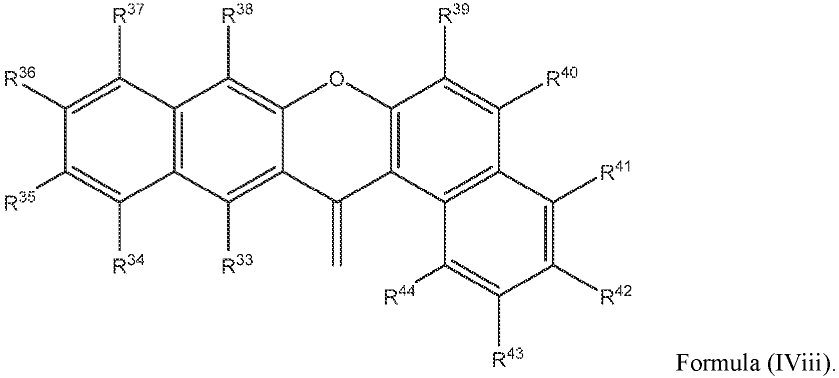

2. The system of claim 1, wherein -
(i) the analyzer is in fluid communication with (a) the inlet stream, (b) the outlet stream, or (c) the inlet stream and the outlet stream of the fluid treatment system,
(ii) the inlet stream, the outlet stream, or the inlet stream and the outlet stream comprises a fluid and the anti-scalant composition,
(iii) the anti-scalant composition comprises (a) a fluorescent tracer, (b) a tagged polymer comprising a monomer tag, or (c) a combination thereof, and
(iv) the fluorometry data collected by the fluorometer comprise (a) an intensity at a fluorescence emission maximum of the fluorescent tracer or the monomer tag at a wavelength pre-selected for the fluorescent tracer or the monomer tag, respectively, (b) a measured amount of the anti-scalant composition, or (c) a combination thereof.

3. The system of claim 1 or 2, wherein the fluid treatment system is a water treatment system.

4. The system of claim 1, wherein the analyzer, the controller, and the pump form an automated control loop configured to (i) collect fluorometry data continuously from the inlet stream and/or the outlet stream of the fluid treatment system, (ii) modify continuously with the pump the amount of the anti-scalant composition that is added to the fluid stream of the fluid treatment system, or (iii) a combination thereof.

5. The system of claim 1, wherein the analyzer, the controller, and the pump form an automated control loop configured to (i) collect fluorometry data intermittently from the inlet stream and/or the outlet stream of the fluid treatment system, (ii) modify intermittently with the pump the amount of the anti-scalant composition that is added to a fluid stream of the fluid treatment system, or (iii) a combination thereof.

6. A method for monitoring and/or controlling dosing of an anti-scalant, the method comprising:
providing (a) the system of any one of claims 1 to 6, or (b) a system comprising -
(i) a fluid treatment system comprising (a) a fluid stream comprising a fluid, (b) an inlet stream, and (c) an outlet stream,
(ii) a reservoir in which the anti-scalant composition of claim 1 is disposed,
(iii) a pump in fluid communication with (a) the reservoir and (b) the fluid stream of the fluid treatment system,
(iv) an analyzer comprising a fluorometer, wherein the fluorometer is in fluid communication with the inlet stream, the outlet stream, or the inlet stream and the outlet stream of the fluid treatment system,
(v) a controller comprising a processor and a memory unit, and
(vi) a dosing algorithm stored by the memory unit of the controller;
adding with the pump an amount of the anti-scalant composition to the fluid stream to create the inlet stream;
analyzing the inlet stream, the outlet stream, or the inlet stream and the outlet stream with the fluorometer to generate fluorometry data comprising (i) a first intensity at a fluorescence emission maximum of the fluorescent tracer or the monomer tag at a wavelength pre-selected for the fluorescent tracer or the monomer tag, respectively, (ii) a measured amount of the anti-scalant composition, or (iii) a combination thereof;
transferring a first signal comprising the fluorometry data to the processor of the controller;
generating with the controller a first dosing instruction by applying the fluorometry data to the dosing algorithm;
transferring a second signal comprising the first dosing instruction to the pump; and
modifying with the pump, based on the first dosing instruction, the amount of the anti-scalant composition that is added to the fluid stream so that a first modified amount of the anti-scalant composition is added to the fluid stream.

7. The method of claim 6, further comprising:
analyzing the inlet stream, the outlet stream, or the inlet stream and the outlet stream with the fluorometer to generate additional fluorometry data comprising (i) a second intensity at the fluorescence emission maximum of the fluorescent tracer or the monomer tag at the wavelength pre-selected for the fluorescent tracer or the monomer tag, respectively, (ii) a second measured amount of the anti-scalant composition, or (iii) a combination thereof;
transferring a third signal comprising the additional fluorometry data to the processor of the controller;
generating with the controller a second dosing instruction by applying the additional fluorometry data to the dosing algorithm;
transferring a fourth signal comprising the second dosing instruction to the pump; and
modifying with the pump, based on the second dosing instruction, the first modified amount of the anti-scalant composition that is added to the fluid stream so that a second modified amount of the anti-scalant composition is added to the fluid stream.

8. The method of claim 6 or 7, wherein the adding of (i) the amount of the anti-scalant composition, (ii) the first modified amount of the anti-scalant composition, (iii) the second modified amount of the anti-scalant composition, or (iv) a combination thereof to the fluid stream is performed continuously.

9. The method of claim 6 or 7, wherein the adding of (i) the amount of the anti-scalant composition, (ii) the first modified amount of the anti-scalant composition, (iii) the second modified amount of the anti-scalant composition, or (iv) a combination thereof to the fluid stream is performed intermittently.

10. The method of claim 6 or 7, wherein the generating of (i) the fluorometry data, (ii) the additional fluorometry data, or (iii) a combination thereof is performed intermittently.

11. The method of claim 6 or 7, wherein (i) the amount of the anti-scalant composition that is added to the fluid stream is less than the first modified amount of the anti-scalant composition that is added to the fluid stream, or (ii) the first modified amount of the anti-scalant composition that is added to the fluid stream is less than the second modified amount of the anti-scalant composition that is added to the fluid stream.

## Patentansprüche

1. System zum Überwachen und/oder Steuern des Dosierens eines Ablagerungen verhütenden Mittels (engl. anti-scalant), wobei das System umfasst:
einen Analysator, umfassend ein Fluorimeter;
eine Steuerung, umfassend einen Prozessor und eine Speichereinheit;
einen Dosieralgorithmus, der von der Speichereinheit der Steuerung gespeichert ist;
eine Pumpe, die ausgestaltet ist, um einem Fluidstrom eines Fluidbehandlungssystems eine Menge einer Ablagerungen verhütenden Zusammensetzung zuzugeben;
und
ein Reservoir in Fluidverbindung mit der Pumpe, wobei sich die Ablagerungen verhütende Zusammensetzung in dem Reservoir befindet;
wobei der Prozessor ausgestaltet ist, um (i) von dem Analysator ein erstes Signal zu empfangen, das Fluorimetriedaten umfasst, die von dem Fluorimeter von (a) einem Einlassstrom, (b) einem Auslassstrom oder (c) einer Kombination davon des Fluidbehandlungssystems gesammelt werden, (ii) eine Dosieranweisung zu bestimmen, indem die Fluorimetriedaten auf den Dosieralgorithmus angewendet werden, und (iii) ein zweites Signal, welches die Dosieranweisung umfasst, der Pumpe zuzuführen,
wobei die Pumpe ausgestaltet ist, um auf Grundlage der Dosieranweisung die Menge der Ablagerungen verhütenden Zusammensetzung, die dem Fluidstrom des Fluidbehandlungssystems zugegeben wird, zu ändern, und
wobei die Ablagerungen verhütende Zusammensetzung eine Polymerzusammensetzung ist, die das markierende Monomer umfasst, wobei die Polymerzusammensetzung umfasst -
ein Copolymer, umfassend -
(i) ein erstes Monomer, ausgewählt aus der Gruppe, die aus (a) einer Verbindung mit der Formel (I), (b) einer Verbindung mit der Formel (II), (c) einer Verbindung oder einem Isomer mit der Formel (III) und (d) einer Verbindung oder einem Isomer mit der Formel (IV) besteht, wobei das erste Monomer das markierende Monomer ist, und
(ii) mindestens ein zweites Monomer, umfassend mindestens eine polymerisierbare Doppelbindung oder mindestens eine polymerisierbare Dreifachbindung, wobei das mindestens eine zweite Monomer ein Ablagerungen hemmendes Monomer ist;
wobei das erste Monomer in dem Copolymer in einer Menge von etwa 0,01 Gew.-% bis etwa 10 Gew.-%, bezogen auf das Gewicht des Copolymers, vorhanden ist; 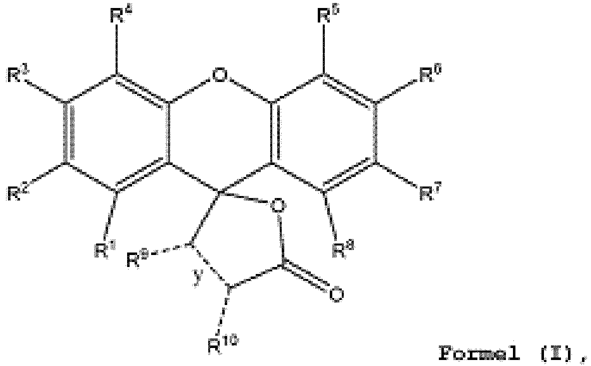 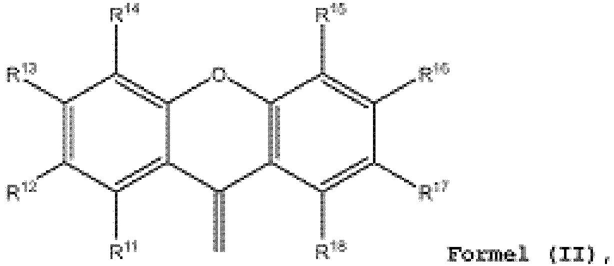 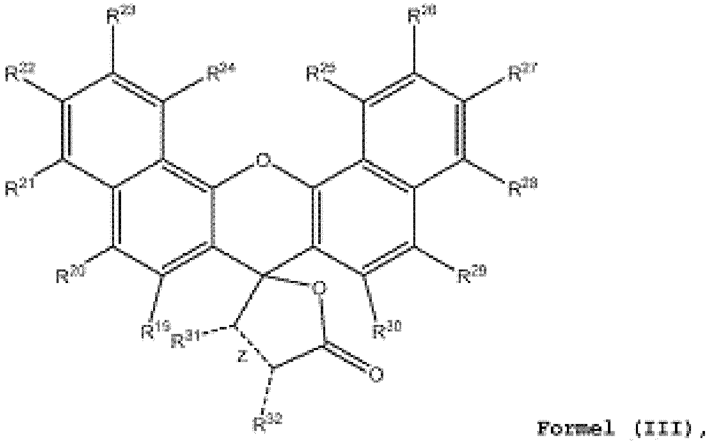 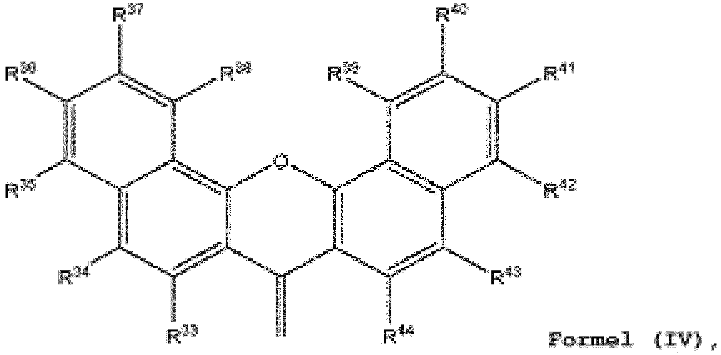
wobei R¹, R², R³, R⁴, R⁵, R⁶, R⁷, R⁸, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹, R³⁰, R³³, R³⁴, R³⁵, R³⁶, R³⁷, R³⁸, R³⁹, R⁴⁰, R⁴¹, R⁴², R⁴³ und R⁴⁴ unabhängig aus der Gruppe ausgewählt sind, die aus Wasserstoff, Hydroxyl, C₁-C₆-Alkoxy, C₂-C₆-Alkenoxy, C₂-C₆-Alkinoxy, -N(R')(R"), C₁-C₆-Alkyl, C₂-C₆-Alkenyl, C₂-C₆-Alkinyl und C₄-C₁₄-Aryl besteht,
wobei R' und R" unabhängig aus der Gruppe ausgewählt sind, die aus Wasserstoff und C₁-C₆-Alkyl besteht,
wobei R⁹, R¹⁰, R³¹ und R³² unabhängig aus der Gruppe ausgewählt sind, die aus Wasserstoff, Hydroxyl, C₁-C₆-Alkyl und C₁-C₆-Alkenyl besteht,
wobei y eine Einfachbindung oder eine Doppelbindung ist,
wobei (i) y eine Doppelbindung ist oder (ii) R⁹ ein C₁-Alkenyl ist,
wobei z eine Einfachbindung oder eine Doppelbindung ist und
wobei (i) z eine Doppelbindung ist oder (ii) R³¹ ein C₁-Alkenyl ist;
wobei die Isomere mit der Formel (III) eine Verbindung mit der Formel (IIIi), eine Verbindung mit der Formel (IIIii), eine Verbindung mit der Formel (IIIiii) oder eine Kombination davon umfassen - 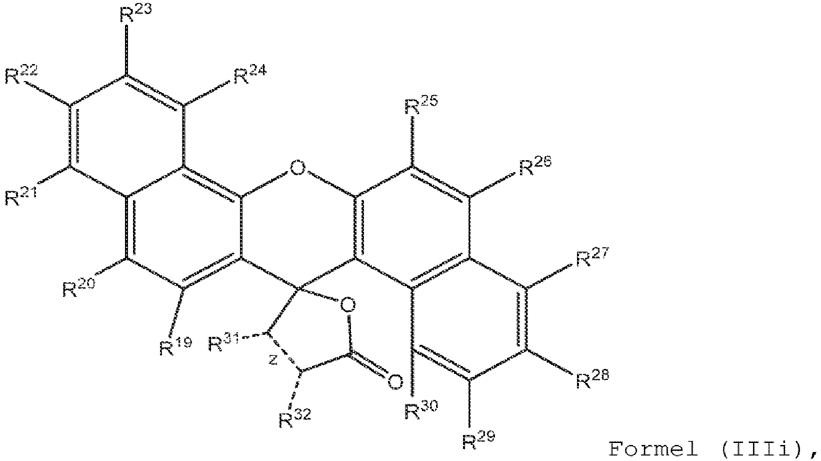 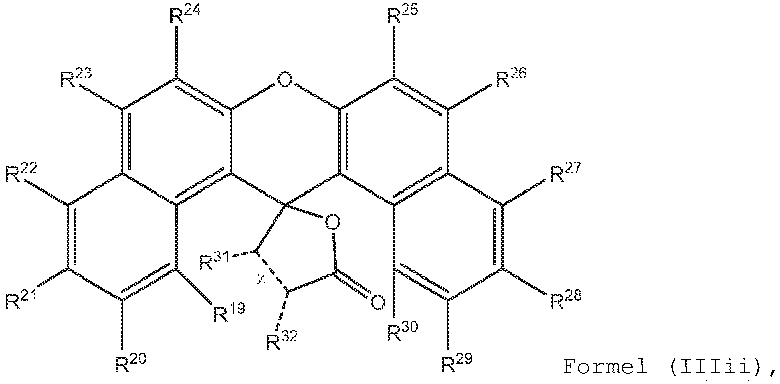 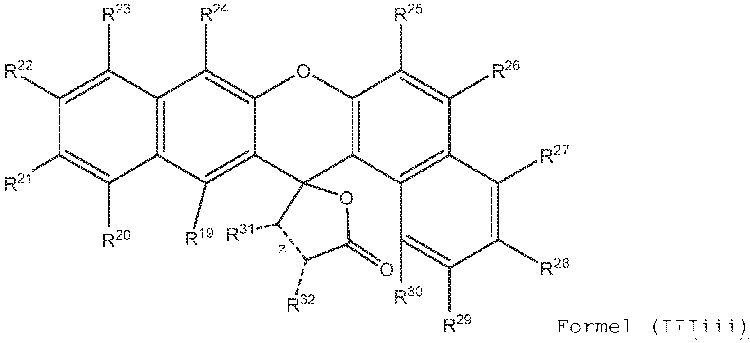 und
wobei die Isomere mit der Formel (IV) eine Verbindung mit der Formel (IVi), eine Verbindung mit der Formel (IVii), eine Verbindung mit der Formel (IViii) oder eine Kombination davon umfassen -

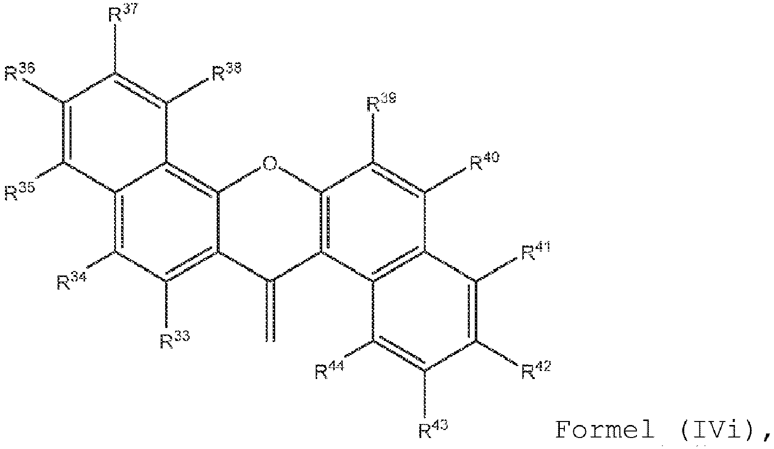

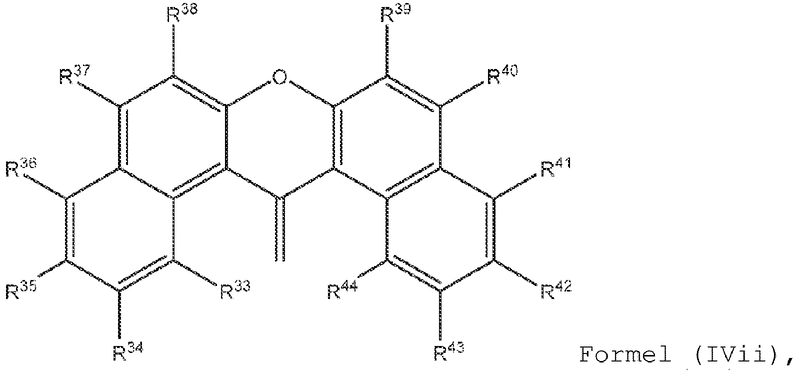

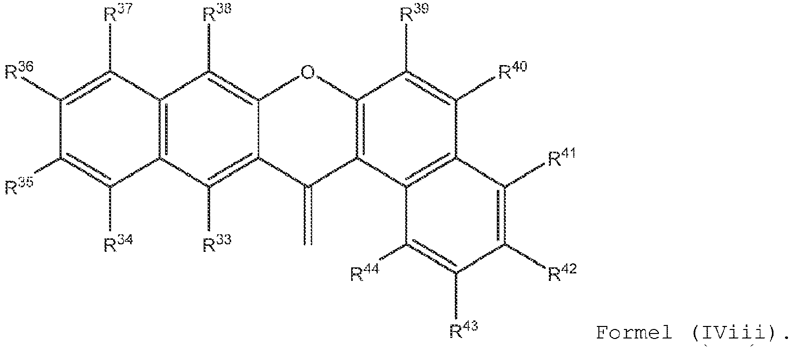

2. System nach Anspruch 1, wobei -
(i) der Analysator in Fluidverbindung mit (a) dem Einlassstrom, (b) dem Auslassstrom oder (c) dem Einlassstrom und dem Auslassstrom des Fluidbehandlungssystems ist,
(ii) der Einlassstrom, der Auslassstrom oder der Einlassstrom und der Auslassstrom ein Fluid und die Ablagerungen verhütende Zusammensetzung umfassen,
(iii) die Ablagerungen verhütende Zusammensetzung (a) einen fluoreszenten Tracer, (b) ein markiertes Polymer, umfassend eine Monomermarkierung, oder (c) eine Kombination davon umfasst und
(iv) die Fluorimetriedaten, die von dem Fluorimeter gesammelt werden, (a) eine Intensität bei einem Fluoreszenzemissionsmaximum des fluoreszenten Tracers oder der Monomermarkierung bei einer Wellenlänge, die für den fluoreszenten Tracer bzw. die Monomermarkierung vorausgewählt werden, (b) eine gemessene Menge der Ablagerungen verhütenden Zusammensetzung oder (c) eine Kombination davon umfassen.

3. System nach Anspruch 1 oder 2, wobei das Fluidbehandlungssystem ein Wasserbehandlungssystem ist.

4. System nach Anspruch 1, wobei der Analysator, die Steuerung und die Pumpe einen automatischen Regelkreis bilden, der ausgestaltet ist, um (i) kontinuierlich Fluorimetriedaten von dem Einlassstrom und/oder dem Auslassstrom des Fluidbehandlungssystems zu sammeln, (ii) kontinuierlich mit der Pumpe die Menge der Ablagerungen verhütenden Zusammensetzung, die dem Fluidstrom des Fluidbehandlungssystems zugegeben wird, zu ändern, oder (iii) eine Kombination davon.

5. System nach Anspruch 1, wobei der Analysator, die Steuerung und die Pumpe einen automatischen Regelkreis bilden, der ausgestaltet ist, um (i) intermittierend Fluorimetriedaten von dem Einlassstrom und/oder dem Auslassstrom des Fluidbehandlungssystems zu sammeln, (ii) intermittierend mit der Pumpe die Menge der Ablagerungen verhütenden Zusammensetzung, die einem Fluidstrom des Fluidbehandlungssystems zugegeben wird, zu ändern, oder (iii) eine Kombination davon.

6. Verfahren zum Überwachen und/oder Steuern des Dosierens eines Ablagerungen verhütenden Mittels, wobei das Verfahren umfasst:
Bereitstellen (a) des Systems nach einem der Ansprüche 1 bis 6 oder (b) eines Systems, umfassend -
(i) ein Fluidbehandlungssystem, umfassend (a) einen Fluidstrom, umfassend ein Fluid, (b) einen Einlassstrom und (c) einen Auslassstrom,
(ii) ein Reservoir, in dem sich die Ablagerungen verhütende Zusammensetzung nach Anspruch 1 befindet,
(iii) eine Pumpe in Fluidverbindung mit (a) dem Reservoir und (b) dem Fluidstrom des Fluidbehandlungssystems,
(iv) einen Analysator, umfassend ein Fluorimeter, wobei das Fluorimeter in Fluidverbindung mit dem Einlassstrom, dem Auslassstrom oder dem Einlassstrom und dem Auslassstrom des Fluidbehandlungssystems ist,
(v) eine Steuerung, umfassend einen Prozessor und eine Speichereinheit, und
(vi) einen Dosieralgorithmus, der von der Speichereinheit der Steuerung gespeichert wird;
Zugeben einer Menge der Ablagerungen verhütenden Zusammensetzung mit der Pumpe zu dem Fluidstrom, um den Einlassstrom zu erzeugen;
Analysieren des Einlassstroms, des Auslassstroms oder des Einlassstroms und des Auslassstroms mit dem Fluorimeter, um Fluorimetriedaten zu erzeugen, umfassend (i) eine erste Intensität bei einem Fluoreszenzemissionsmaximum des fluoreszenten Tracers oder der Monomermarkierung bei einer Wellenlänge, die für den fluoreszenten Tracer bzw. die Monomermarkierung vorausgewählt wird, (ii) eine gemessene Menge der Ablagerungen verhütenden Zusammensetzung oder (iii) eine Kombination davon;
Übermitteln eines ersten Signals, umfassend die Fluorimetriedaten, an den Prozessor der Steuerung;
Erzeugen einer ersten Dosieranweisung mit der Steuerung durch Anwenden der Fluorimetriedaten auf den Dosieralgorithmus;
Übermitteln eines zweiten Signals, umfassend die erste Dosieranweisung, an die Pumpe; und
Ändern mit der Pumpe, auf Grundlage der ersten Dosieranweisung, der Menge der Ablagerungen verhütenden Zusammensetzung, die dem Fluidstrom zugegeben wird, sodass dem Fluidstrom eine erste geänderte Menge der Ablagerungen verhütenden Zusammensetzung zugegeben wird.

7. Verfahren nach Anspruch 6, ferner umfassend:
Analysieren des Einlassstroms, des Auslassstroms oder des Einlassstroms und des Auslassstroms mit dem Fluorimeter, um zusätzliche Fluorimetriedaten zu erzeugen, umfassend (i) eine zweite Intensität bei dem Fluoreszenzemissionsmaximum des fluoreszenten Tracers oder der Monomermarkierung bei der Wellenlänge, die für den fluoreszenten Tracer bzw. die Monomermarkierung vorausgewählt wird, (ii) eine zweite gemessene Menge der Ablagerungen verhütenden Zusammensetzung oder (iii) eine Kombination davon;
Übermitteln eines dritten Signals, umfassend die zusätzlichen Fluorimetriedaten, an den Prozessor der Steuerung;
Erzeugen einer zweiten Dosieranweisung mit der Steuerung durch Anwenden der zusätzlichen Fluorimetriedaten auf den Dosieralgorithmus;
Übermitteln eines vierten Signals, umfassend die zweite Dosieranweisung, an die Pumpe; und
Ändern mit der Pumpe, auf Grundlage der zweiten Dosieranweisung, der ersten geänderten Menge der Ablagerungen verhütenden Zusammensetzung, die dem Fluidstrom zugegeben wird, sodass dem Fluidstrom eine zweite geänderte Menge der Ablagerungen verhütenden Zusammensetzung zugegeben wird.

8. Verfahren nach Anspruch 6 oder 7, wobei das Zugeben von (i) der Menge der Ablagerungen verhütenden Zusammensetzung, (ii) der ersten geänderten Menge der Ablagerungen verhütenden Zusammensetzung, (iii) der zweiten geänderten Menge der Ablagerungen verhütenden Zusammensetzung oder (iv) einer Kombination davon zu dem Fluidstrom kontinuierlich durchgeführt wird.

9. Verfahren nach Anspruch 6 oder 7, wobei das Zugeben von (i) der Menge der Ablagerungen verhütenden Zusammensetzung, (ii) der ersten geänderten Menge der Ablagerungen verhütenden Zusammensetzung, (iii) der zweiten geänderten Menge der Ablagerungen verhütenden Zusammensetzung oder (iv) einer Kombination davon zu dem Fluidstrom intermittierend durchgeführt wird.

10. Verfahren nach Anspruch 6 oder 7, wobei das Erzeugen (i) der Fluorimetriedaten, (ii) der zusätzlichen Fluorimetriedaten oder (iii) einer Kombination davon intermittierend durchgeführt wird.

11. Verfahren nach Anspruch 6 oder 7, wobei (i) die Menge der Ablagerungen verhütenden Zusammensetzung, die dem Fluidstrom zugegeben wird, kleiner ist als die erste geänderte Menge der Ablagerungen verhütenden Zusammensetzung, die dem Fluidstrom zugegeben wird, oder (ii) die erste geänderte Menge der Ablagerungen verhütenden Zusammensetzung, die dem Fluidstrom zugegeben wird, geringer ist als die zweite geänderte Menge der Ablagerungen verhütenden Zusammensetzung, die dem Fluidstrom zugegeben wird.

## Revendications

1. Système de surveillance et/ou de commande du dosage d'un agent anti-tartre, le système comprenant :
un analyseur comprenant un fluoromètre ;
un dispositif de commande comprenant un processeur et une unité de mémoire ;
un algorithme de dosage stocké par l'unité de mémoire du dispositif de commande ;
une pompe configurée pour ajouter une quantité d'une composition anti-tartre dans un flux de fluide d'un système de traitement de fluide ; et
un réservoir en communication fluidique avec la pompe, dans lequel la composition anti-tartre est disposée dans le réservoir ;
dans lequel le processeur est configuré pour (i) recevoir à partir de l'analyseur un premier signal comprenant des données de fluorométrie collectées par le fluoromètre à partir de (a) un flux d'entrée, (b) un flux de sortie, ou (c) une combinaison de ceux-ci du système de traitement de fluide, (ii) déterminer une instruction de dosage en appliquant les données de fluorométrie à l'algorithme de dosage, et (iii) fournir un deuxième signal comprenant l'instruction de dosage à la pompe,
dans lequel la pompe est configurée pour modifier, sur la base de l'instruction de dosage, la quantité de la composition anti-tartre qui est ajoutée au flux de fluide du système de traitement de fluide, et
dans lequel la composition anti-tartre est une composition polymère comprenant le monomère de marquage, dans lequel la composition polymère comprend un copolymère comprenant
(i) un premier monomère choisi dans le groupe constitué par (a) un composé de formule (I), (b) un composé de formule (II), (c) un composé ou un isomère de formule (III), et (d) un composé ou un isomère de formule (IV), dans lequel le premier monomère est le monomère de marquage, et
(ii) au moins un deuxième monomère comprenant au moins une double liaison polymérisable ou au moins une triple liaison polymérisable, dans lequel ledit au moins un deuxième monomère est un monomère inhibant le tartre ;
dans lequel le premier monomère est présent dans le copolymère en une quantité d'environ 0,01 % à environ 10 %, en poids, sur la base du poids du copolymère ; 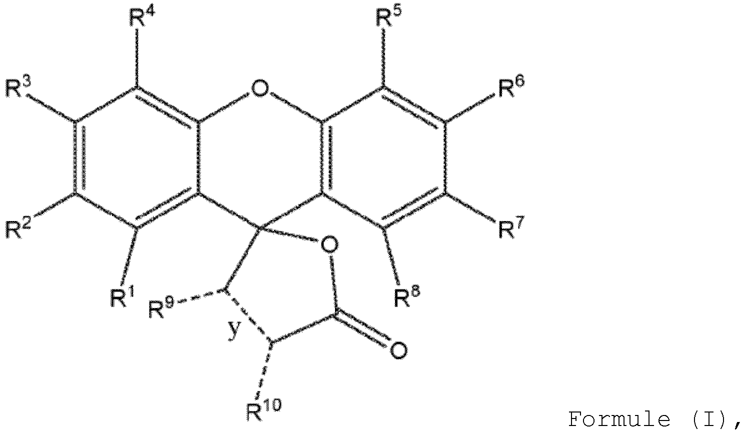 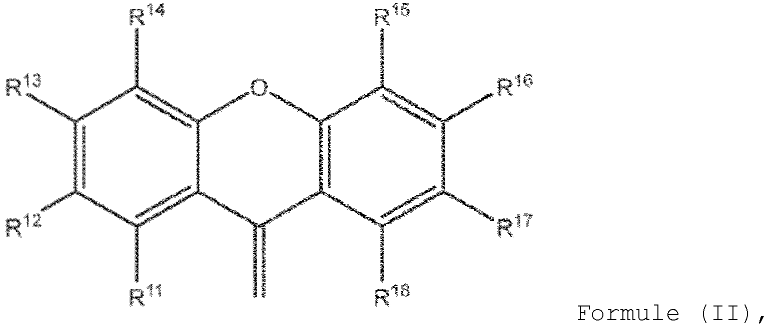 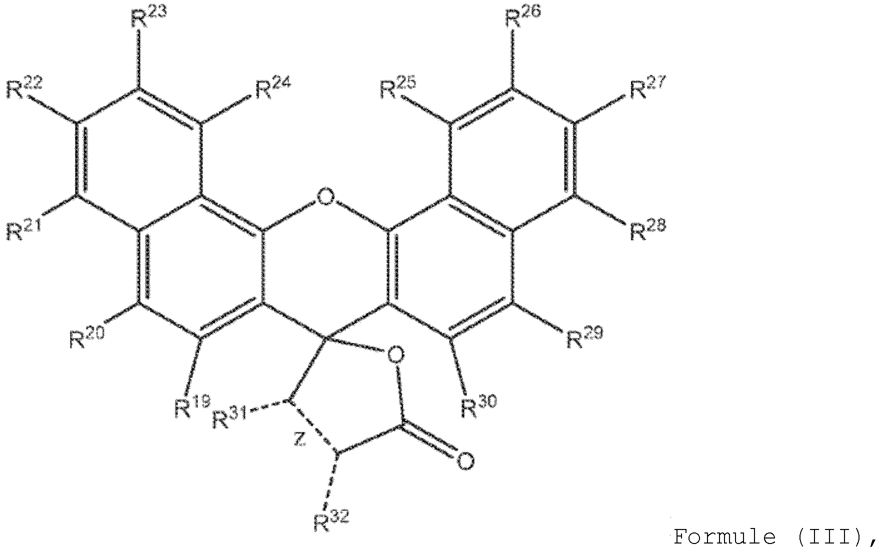 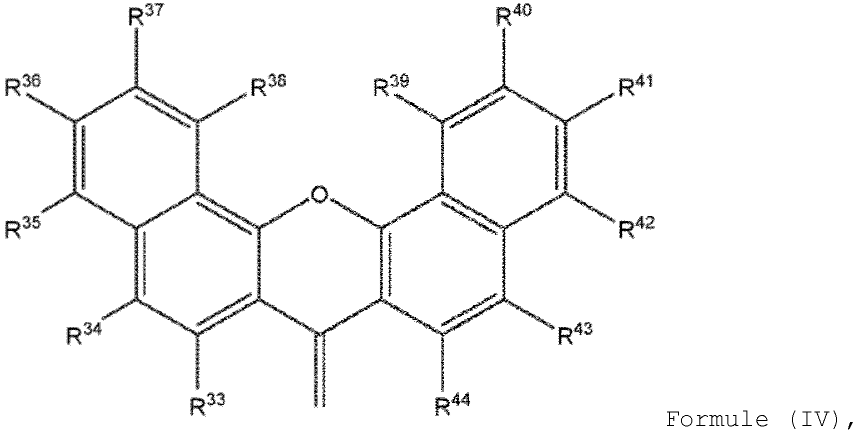
dans lequel R¹, R², R³, R⁴, R⁵, R⁶, R⁷, R⁸, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹, R³⁰, R³³, R³⁴, R³⁵, R³⁶, R³⁷, R³⁸, R³⁹, R⁴⁰, R⁴¹, R⁴², R⁴³ et R⁴⁴ sont indépendamment choisis dans le groupe constitué par un atome d'hydrogène, un groupe hydroxyle, un groupe alcoxy en C₁ à C₆, un groupe alcénoxy en C₂ à C₆, un groupe alcynoxy en C₂ à C₆, -N(R')(R"), un groupe alkyle en C₁ à C₆, un groupe alcényle en C₂ à C₆, un groupe alcynyle en C₂ à C₆ et un groupe aryle en C₄ à C₁₄,
dans lequel R' et R" sont indépendamment choisis dans le groupe constitué par un atome d'hydrogène et un groupe alkyle en C₁ à C₆,
dans lequel R⁹, R¹⁰, R³¹ et R³² sont indépendamment choisis dans le groupe constitué par un atome d'hydrogène, un groupe hydroxyle, un groupe alkyle en C₁ à C₆ et un groupe alcényle en C₁ à C₆,
dans lequel y est une liaison simple ou une double liaison,
dans lequel (i) y est une double liaison ou (ii) R⁹ est un groupe alcényle en C₁,
dans lequel z est une liaison simple ou une double liaison, et
dans lequel (i) z est une double liaison ou (ii) R³¹ est un groupe alcényle en C₁ ;
dans lequel les isomères de formule (III) comprennent un composé de formule (IIIi), un composé de formule (IIIii), un composé de formule (IIIiii), ou une combinaison de ceux-ci 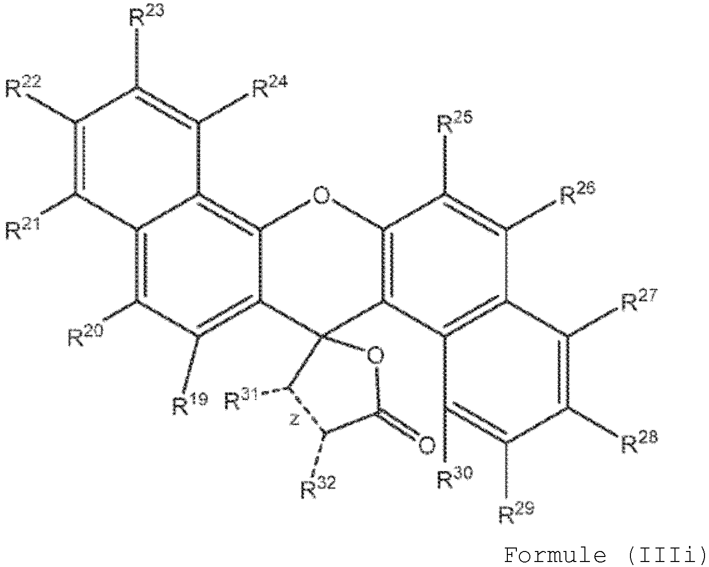 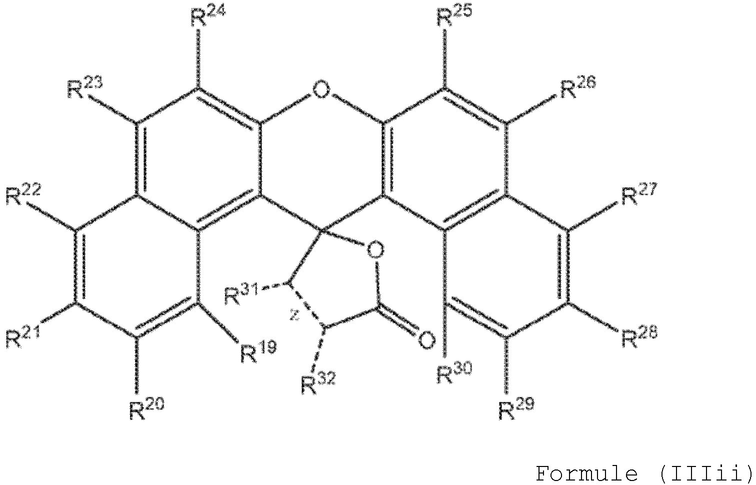 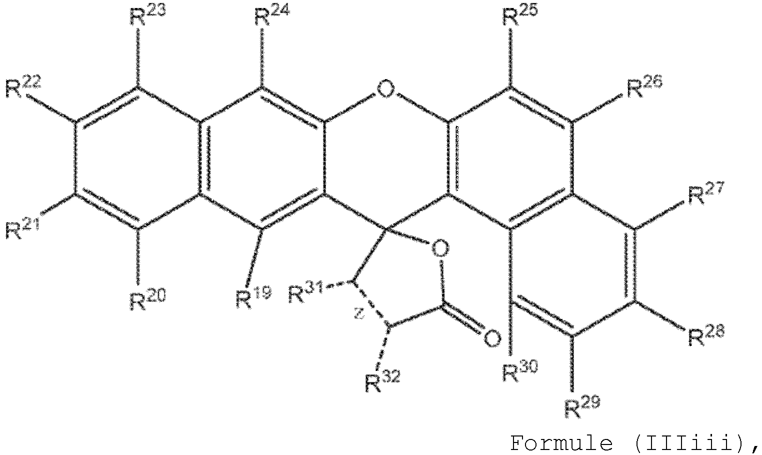 et
dans lequel les isomères de formule (IV) comprennent un composé de formule (IVi), un composé de formule (IVii), un composé de formule (IViii), ou une combinaison de ceux-ci

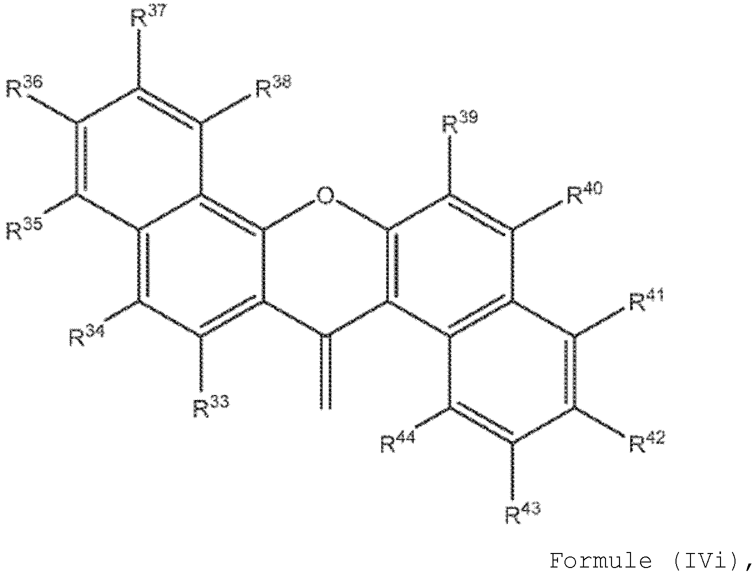

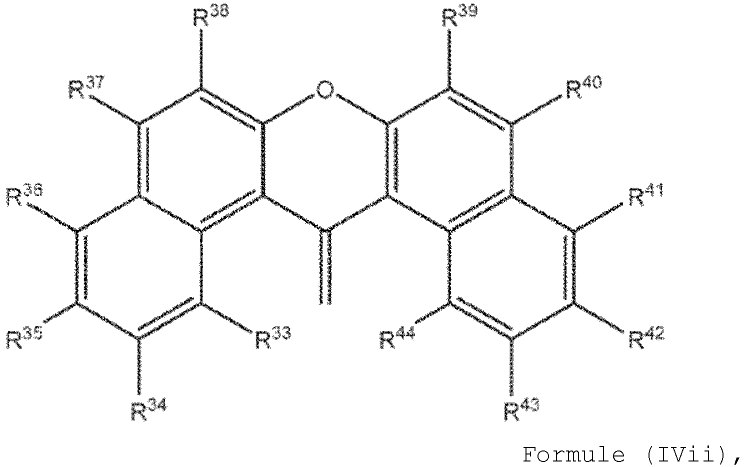

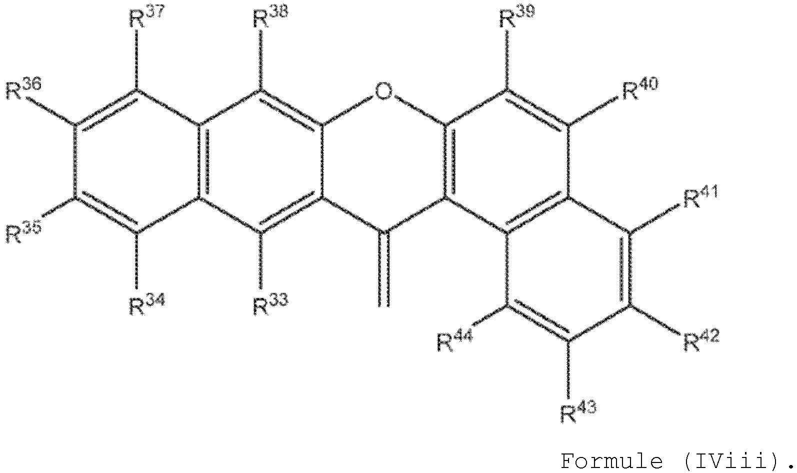

2. Système selon la revendication 1, dans lequel
(i) l'analyseur est en communication fluidique avec (a) le flux d'entrée, (b) le flux de sortie ou (c) le flux d'entrée et le flux de sortie du système de traitement de fluide,
(ii) le flux d'entrée, le flux de sortie ou le flux d'entrée et le flux de sortie comprend un fluide et la composition anti-tartre,
(iii) la composition anti-tartre comprend (a) un traceur fluorescent, (b) un polymère marqué comprenant un marqueur monomère, ou (c) une combinaison de ceux-ci, et
(iv) les données de fluorométrie collectées par le fluoromètre comprennent (a) une intensité à une émission de fluorescence maximale du traceur fluorescent ou du marqueur monomère à une longueur d'onde présélectionnée pour le traceur fluorescent ou le marqueur monomère, respectivement, (b) une quantité mesurée de la composition anti-tartre, ou (c) une combinaison de celles-ci.

3. Système selon la revendication 1 ou 2, dans lequel le système de traitement de fluide est un système de traitement de l'eau.

4. Système selon la revendication 1, dans lequel l'analyseur, le dispositif de commande et la pompe forment une boucle de commande automatisée configurée pour (i) collecter des données de fluorométrie en continu à partir du flux d'entrée et/ou du flux de sortie du système de traitement de fluide, (ii) modifier en continu avec la pompe la quantité de la composition anti-tartre qui est ajoutée au flux de fluide du système de traitement de fluide, ou (iii) une combinaison de ceux-ci.

5. Système selon la revendication 1, dans lequel l'analyseur, le dispositif de commande et la pompe forment une boucle de commande automatisée configurée pour (i) collecter des données de fluorométrie par intermittence à partir du flux d'entrée et/ou du flux de sortie du système de traitement de fluide, (ii) modifier par intermittence avec la pompe la quantité de la composition anti-tartre qui est ajoutée à un flux de fluide du système de traitement de fluide, ou (iii) une combinaison de ceux-ci.

6. Procédé de surveillance et/ou de commande du dosage d'un agent anti-tartre, le procédé comprenant :
la fourniture (a) du système selon l'une quelconque des revendications 1 à 6, ou (b) d'un système comprenant
(i) un système de traitement de fluide comprenant (a) un flux de fluide comprenant un fluide, (b) un flux d'entrée et (c) un flux de sortie,
(ii) un réservoir dans lequel est disposée la composition anti-tartre selon la revendication 1,
(iii) une pompe en communication fluidique avec (a) le réservoir et (b) le flux de fluide du système de traitement de fluide,
(iv) un analyseur comprenant un fluoromètre, dans lequel le fluoromètre est en communication fluidique avec le flux d'entrée, le flux de sortie ou le flux d'entrée et le flux de sortie du système de traitement de fluide,
(v) un dispositif de commande comprenant un processeur et une unité de mémoire, et
(vi) un algorithme de dosage stocké par l'unité de mémoire du dispositif de commande ;
l'ajout avec la pompe d'une quantité de la composition anti-tartre au flux de fluide pour créer le flux d'entrée ;
l'analyse du flux d'entrée, du flux de sortie ou du flux d'entrée et du flux de sortie avec le fluorimètre pour générer des données de fluorométrie comprenant (i) une première intensité à une émission de fluorescence maximale du traceur fluorescent ou du marqueur monomère à une longueur d'onde présélectionnée pour le traceur fluorescent ou le marqueur monomère, respectivement, (ii) une quantité mesurée de la composition anti-tartre, ou (iii) une combinaison de celles-ci ;
le transfert d'un premier signal comprenant les données de fluorométrie au processeur du dispositif de commande ;
la génération avec le dispositif de commande d'une première instruction de dosage en appliquant les données de fluorométrie à l'algorithme de dosage ;
le transfert d'un deuxième signal comprenant la première instruction de dosage à la pompe ; et
la modification avec la pompe, sur la base de la première instruction de dosage, de la quantité de la composition anti-tartre qui est ajoutée au flux de fluide de sorte qu'une première quantité modifiée de la composition anti-tartre est ajoutée au flux de fluide.

7. Procédé selon la revendication 6, comprenant en outre :
l'analyse du flux d'entrée, du flux de sortie ou du flux d'entrée et du flux de sortie avec le fluorimètre pour générer des données de fluorométrie supplémentaires comprenant (i) une deuxième intensité à une émission de fluorescence maximale du traceur fluorescent ou du marqueur monomère à la longueur d'onde présélectionnée pour le traceur fluorescent ou le marqueur monomère, respectivement, (ii) une deuxième quantité mesurée de la composition anti-tartre, ou (iii) une combinaison de celles-ci ;
le transfert d'un troisième signal comprenant les données de fluorométrie supplémentaires au processeur du dispositif de commande ;
la génération avec le dispositif de commande d'une deuxième instruction de dosage en appliquant les données de fluorométrie supplémentaires à l'algorithme de dosage ;
le transfert d'un quatrième signal comprenant la deuxième instruction de dosage à la pompe ; et
la modification avec la pompe, sur la base de la deuxième instruction de dosage, de la première quantité modifiée de la composition anti-tartre qui est ajoutée au flux de fluide de sorte qu'une deuxième quantité modifiée de la composition anti-tartre est ajoutée au flux de fluide.

8. Procédé selon la revendication 6 ou 7, dans lequel l'ajout de (i) la quantité de la composition anti-tartre, (ii) la première quantité modifiée de la composition anti-tartre, (iii) la deuxième quantité modifiée de la composition anti-tartre, ou (iv) une combinaison de celles-ci au flux de fluide est effectué en continu.

9. Procédé selon la revendication 6 ou 7, dans lequel l'ajout de (i) la quantité de la composition anti-tartre, (ii) la première quantité modifiée de la composition anti-tartre, (iii) la deuxième quantité modifiée de la composition anti-tartre, ou (iv) une combinaison de celles-ci au flux de fluide est effectué par intermittence.

10. Procédé selon la revendication 6 ou 7, dans lequel la génération (i) des données de fluorométrie, (ii) des données de fluorométrie supplémentaires, ou (iii) d'une combinaison de celles-ci est effectuée par intermittence.

11. Procédé selon la revendication 6 ou 7, dans lequel (i) la quantité de la composition anti-tartre qui est ajoutée au flux de fluide est inférieure à la première quantité modifiée de la composition anti-tartre qui est ajoutée au flux de fluide ou (ii) la première quantité modifiée de la composition anti-tartre qui est ajoutée au flux de fluide est inférieure à la deuxième quantité modifiée de la composition anti-tartre qui est ajoutée au flux de fluide.
